(19) Europäisches Patentamt / European Patent Office / Office européen des brevets

(11) **EP 3 071 948 B1**

(12) **FASCICULE DE BREVET EUROPEEN**

(45) Date de publication et mention de la délivrance du brevet:
**18.10.2017 Bulletin 2017/42**

(21) Numéro de dépôt: **14802429.2**

(22) Date de dépôt: **21.11.2014**

(51) Int Cl.:
*G01N 21/64* $^{(2006.01)}$

(86) Numéro de dépôt international:
**PCT/EP2014/075336**

(87) Numéro de publication internationale:
**WO 2015/075209 (28.05.2015 Gazette 2015/21)**

(54) **PROCEDE DE DETECTION D'UNE ESPECE FLUORESCENTE REVERSIBLEMENT PHOTOCONVERTIBLE UTILISANT L'IMAGERIE HORS PHASE APRES MODULATION OPTIQUE**

VERFAHREN ZUR DETEKTION EINES PHOTOSCHALTBAREN FLUORESZIERENDEN MATERIALS UNTER VERWENDUNG VON PHASENVERSETZTER BILDGEBUNG NACH OPTISCHER MODULATION

METHOD OF DETECTING A PHOTOSWITCHABLE FLUORESCENT SPECIES USING OUT-OF-PHASE IMAGING AFTER OPTICAL MODULATION

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **21.11.2013 FR 1361476**

(43) Date de publication de la demande:
**28.09.2016 Bulletin 2016/39**

(73) Titulaires:
• **Centre National de la Recherche Scientifique**
  **75016 Paris (FR)**
• **Institut Curie**
  **75005 Paris (FR)**
• **Ecole Normale Supérieure de Paris**
  **75005 Paris Cedex 05 (FR)**
• **Université Pierre et Marie Curie**
  **75252 Paris Cedex 05 (FR)**

(72) Inventeurs:
• **JULLIEN, Ludovic**
  **F-94110 Arcueil (FR)**
• **LE SAUX, Thomas**
  **F-75020 Paris (FR)**
• **GAUTIER, Arnaud**
  **F-75013 Paris (FR)**
• **CROQUETTE, Vincent**
  **F-92160 Antony (FR)**
• **SARANG, Nath**
  **New Delhi 110 016 (IN)**
• **WANG, Pencheng**
  **63108 Saint Louis (US)**
• **QUERARD, Jérôme**
  **F-92120 Montrouge (FR)**
• **ALBRIGHT, Samantha**
  **Boston, MA 02215 (US)**

(74) Mandataire: **Priori, Enrico et al**
**Marks & Clerk France**
**Conseils en Propriété Industrielle**
**Immeuble «Visium»**
**22, avenue Aristide Briand**
**94117 Arcueil Cedex (FR)**

(56) Documents cités:
**WO-A1-01/27883    GB-A- 2 089 029**

• **PENGCHENG WANG ET AL: "Photochemical properties of Spinach and its use in selective imaging", CHEMICAL SCIENCE, vol. 4, no. 7, 1 janvier 2013 (2013-01-01) , page 2865, XP055131010, ISSN: 2041-6520, DOI: 10.1039/c3sc50729g**

**Description**

L'invention porte sur un procédé de détection d'une espèce fluorescente réversiblement photoconvertible. Un tel procédé présente de nombreuses applications, notamment en chimie, en biologie et dans le domaine des mesures environnementales.

On entend par « espèce » une espèce chimique telle qu'une molécule ou un complexe, ou un objet physique tel qu'une nanoparticule. On entend par « espèce réversiblement photoconvertible » (« photoswitchable » dans la littérature en anglais) une espèce présentant au moins deux états distincts, ayant des propriétés de fluorescence différentes, et pouvant passer d'un état à l'autre de manière réversible par effet de la lumière. Des exemples d'espèces fluorescentes réversiblement photoconvertibles sont la protéine « Dronpa » et le complexe « Spinach - DFHBI » (« Spinach » étant un aptamère d'ARN et DFHBI une sonde fluorogène). Ces espèces peuvent, en particulier, être utilisées comme sondes ou marqueurs.

L'imagerie, et plus particulièrement la microscopie par fluorescence est devenue un outil indispensable en biologie, mais également dans d'autres disciplines telles que la science des matériaux. Ses applications, cependant, sont limitées par la capacité d'observer un signal d'intérêt dans un fond de fluorescence ou de bruit. Ce problème est particulièrement aigu dans les applications d'imagerie in vivo, dans lesquelles les marqueurs fluorescents à détecter sont dispersés dans un milieu complexe auto-fluorescent et/ou diffusant ; le signal utile se retrouve alors noyé dans un bruit de fond intense.

Une autre limite des techniques de détection et imagerie par fluorescence conventionnelles réside dans le fait que de nombreux fluorophores présentent des bandes d'émission larges ; dès lors, il est difficile de détecter sélectivement plusieurs marqueurs fluorescents dans un même échantillon, car leurs spectres d'émission tendent à se superposer.

Pour surmonter ces limites, il a été proposé d'utiliser des sondes fluorescentes réversiblement photoconvertibles, un éclairage d'excitation de la fluorescence modulé (variable dans le temps de façon prédéterminée) et une démodulation du signal de fluorescence détecté. Cela permet d'exploiter la dynamique temporelle d'une sonde réversiblement photoconvertible - qui lui est propre et est différente de celle de fluorophores interférents - pour extraire un signal utile du bruit de fond ; on parle alors de « contraste dynamique ».

Une technique connue de l'art antérieur exploitant ce principe est connue sous le nom de OLID, acronyme de «Optical Lock-In Detection », c'est-à-dire « détection synchrone optique ». Elle est décrite dans l'article de G. Marriott et al. « Optical lock-in detection imaging microscopy for contrast-enhanced imaging in living cell », PNAS, vol. 105, n°46, pages 17789-17794 (18 novembre 2008). Un inconvénient de cette technique est de ne pas fournir d'information quantitative sur la concentration du fluorophore réversiblement photoconvertible. Elle nécessite de plus une séquence d'excitation lumineuse à deux couleurs et au moins un pixel de référence.

Une autre technique connue de l'art antérieur utilisant une sonde fluorescente photoconvertible et une excitation modulée est connue sous le nom de SAFIRe, acronyme de « Synchronously Amplified Fluorescence Image Recovery » c'est-à-dire « récupération d'image par fluorescence amplifiée synchroniquement ». Elle est décrite dans l'article de Ch. I. Richards et al. « Synchronously Amplified Fluorescence Image Recovery (SAFIRe) », J. Phys. Chem. B 2010, 114, 660 - 665. Cette technique utilise, elle aussi, une excitation à deux couleurs. L'optimisation du contraste dynamique présente l'inconvénient d'être fait de manière empirique, ce qui introduit une complexité de mise en oeuvre additionnelle.

L'article de Q. Wei et A. Wei « Optical Imaging with Dynamic Contrast Agents », Chem. Eur. J., 17, 1080 - 1091 présente plusieurs techniques de contraste dynamique connues. Outre les techniques précitées OLID et SAFIRe, basées sur une modulation optique de la fluorescence, cet article décrit des techniques exploitant une modulation magnétomotive ou photothermique. Ces techniques sont complexes à mettre en oeuvre, justement car elles nécessitent à la fois un système optique pour l'excitation et la détection de la fluorescence et un système de modulation non optique (magnétique ou thermique).

Ainsi, toutes les techniques de détection de fluorescence connues de l'art antérieur exploitant un contraste dynamique présentent l'inconvénient d'une mise en oeuvre relativement complexe. En outre, aucune d'entre elles ne présente une sélectivité suffisante pour permettre la détection successive d'un nombre important (de l'ordre de 10, voire davantage) d'espèces fluorescentes dans un même échantillon. Par ailleurs, ces techniques ont été développées exclusivement pour des applications microscopiques et ne peuvent pas être transposées aisément à la télédétection d'espèces fluorescentes dans l'environnement.

L'invention vise à surmonter au moins un des inconvénients précités de l'art antérieur.

Un objet de l'invention permettant d'atteindre ce but est un procédé de détection d'au moins une espèce fluorescente réversiblement photoconvertible, comportant les étapes suivantes :

a) éclairer un échantillon contenant ladite ou au moins une dite espèce fluorescente réversiblement photoconvertible avec une lumière d'éclairage modulée périodiquement ; et

b) détecter un rayonnement de fluorescence émis par ledit échantillon ainsi éclairé ;

caractérisé en ce qu'il comprend également l'étape suivante :

c) extraire l'amplitude de la composante de l'intensité dudit rayonnement de fluorescence présentant la même périodicité que ladite lumière d'éclairage modulée périodiquement et en quadrature de phase par rapport à elle ;

et en ce que l'intensité moyenne de ladite lumière d'éclairage et sa fréquence de modulation sont choisies de manière à maximiser ladite amplitude de la composante d'intensité dudit rayonnement de fluorescence.

Selon différents modes de réalisation de l'invention :

- au moins une dite espèce fluorescente réversiblement photoconvertible (P) présente un premier et un deuxième état chimique, au moins un desdits états étant fluorescent, ladite ou chaque dite espèce fluorescente réversiblement photoconvertible (P) étant susceptible d'être convertie dudit premier état audit deuxième état par une première réaction photo-induite, puis de retourner audit premier état à la fois par une réaction thermo-induite et par une seconde réaction photo-induite et ladite lumière d'éclairage peut présenter une intensité moyenne I$^0$ et être modulée à une fréquence f avec :

$$I^0 = \frac{k_{21}^\Delta}{\sigma_{12} + \sigma_{21}}$$

$$f = k_{21}^\Delta/\pi$$

où :

- $\sigma_{12}I^0$ et $\sigma_{21}I^0$ sont, respectivement, les constantes cinétiques de ladite première réaction photo-induite de ladite espèce fluorescente et de ladite seconde réaction photo-induite de ladite espèce fluorescente ; et
- $k_{21}^\Delta$ est la constante cinétique de ladite réaction thermo-induite de ladite espèce fluorescente.

- Lors de ladite étape a), ledit échantillon peut être éclairé par une lumière d'éclairage sensiblement monochromatique.
- Ladite lumière d'éclairage (FEX) comprend une première lumière d'éclairage sensiblement monochromatique (FEX1) de longueur d'onde $\lambda_1$ et une seconde lumière d'éclairage sensiblement monochromatique (FEX2), de longueur d'onde $\lambda_2$, différente de $\lambda_1$ la première et la seconde dites lumières d'éclairage étant adaptées à induire la photoconversion desdits états d'au moins une dite espèce fluorescente réversiblement photoconvertible (P) et dont au moins la première dite lumière d'éclairage est modulée périodiquement.
- Au moins une dite espèce fluorescente réversiblement photoconvertible (P) présente un premier et un deuxième état chimique, au moins un desdits états étant fluorescent, ladite ou chaque dite espèce fluorescente réversiblement photoconvertible (P) étant susceptible d'être convertie dudit premier état audit deuxième état par une première réaction photo-induite, puis de retourner audit premier état par une seconde réaction photo-induite, et dans lequel ladite première lumière d'éclairage présente une intensité moyenne $I_1^0$ et est modulée à une fréquence f' et ladite seconde lumière d'éclairage présente une intensité sensiblement constante $I_2^0$ avec:

$$\frac{I_2^0}{I_1^0} = \frac{\sigma_{12,1} + \sigma_{21,1}}{\sigma_{12,2} + \sigma_{21,2}}$$

$$\frac{f'}{I_1^0} = \left(\sigma_{12,1} + \sigma_{21,1}\right)/\pi$$

où:

- $\sigma_{12,1}I_1^0$ et $\sigma_{21,1}I_1^0$ sont, respectivement, les constantes cinétiques de ladite première et de ladite seconde réaction photo-induite par ladite première lumière d'éclairage ; et
- $\sigma_{12,2}I_2^0$ et $\sigma_{21,2}I_2^0$ sont, respectivement, les constantes cinétiques de ladite première et de ladite seconde réaction photo-induite par ladite seconde lumière d'éclairage.

- Ledit échantillon peut contenir une pluralité de dites espèces fluorescentes réversiblement photoconvertibles présentant des propriétés dynamiques différentes, lesdites étapes a) à c) étant mises en oeuvre successivement pour la détection d'au moins deux dites espèces fluorescentes réversiblement photoconvertibles.
- Lesdites étapes b) et c) peuvent être mises en oeuvre par détection synchrone dudit rayonnement de fluorescence.
- Ledit échantillon peut contenir au moins une autre espèce fluorescente.
- Le procédé peut comporter également l'étape suivante :

  d) déterminer la concentration de ladite ou d'au moins une dite espèce fluorescente réversiblement photoconvertible à partir de la composante de l'intensité dudit rayonnement de fluorescence extraite lors de ladite étape c).

- Ladite ou au moins une dite espèce fluorescente réversiblement photoconvertible peut être choisie parmi : une protéine fluorescente photochrome ; et un complexe d'une biomolécule, comme par exemple un aptamère ou une protéine, avec une sonde fluorogène.
- Ledit échantillon peut comporter de la matière biologique.

Un autre objet de l'invention est un procédé de microscopie à fluorescence mettant en oeuvre un tel procédé de détection.

Un autre objet de l'invention est un procédé de télédétection optique mettant en oeuvre un tel procédé de détection.

Selon des modes de réalisation de l'invention :

- ledit échantillon peut comprendre un organisme vivant, et au moins un élément choisi parmi la présence et la concentration d'une dite espèce fluorescente réversiblement photoconvertible (P) peut être mesuré à partir de la composante de l'intensité dudit rayonnement de fluorescence extraite lors de ladite étape c) sans effectuer de prélèvement sur ledit organisme vivant.
- ladite lumière d'éclairage (FEX) est émise selon une direction et ladite modulation périodique de ladite lumière d'éclairage (FEX) est mise en oeuvre par une modulation de ladite direction d'émission de ladite lumière d'éclairage (FEX).

Selon un autre mode de réalisation, l'invention est un procédé dans lequel ladite lumière d'éclairage (FEX) comprend une partie de la lumière du jour et dans lequel ladite partie de la lumière du jour participe à l'intensité lumineuse reçue par ladite espèce fluorescente réversiblement photoconvertible (P) en restant inférieure ou égale à la dite intensité $I^0$.

D'autres caractéristiques, détails et avantages de l'invention ressortiront à la lecture de la description faite en référence aux figures annexées données à titre d'exemple, dans lesquelles:

- la figure 1 illustre le principe général d'un procédé selon un mode de réalisation de l'invention ;
- les figures 2A et 2B sont des graphiques illustrant comment les composantes en phase et en quadrature de l'intensité de fluorescence dépendent des paramètres dynamiques d'une espèce fluorescente réversiblement photoconvertible ;
- les figures 3A, 3B, 3C, 3D, 3E et 3F sont des graphiques illustrant le résultat de calculs de l'amplitude normalisée en quadrature de phase, en fonction de différents paramètres de contrôle.
- la figure 4 est un graphique illustrant l'application d'un procédé selon un mode de réalisation de l'invention à la détermination de la concentration d'une espèce fluorescente réversiblement photoconvertible ;
- les figures 5A et 5B sont des graphiques illustrant les calculs effectués pour optimiser la détection en quadrature de phase dans le cas d'une excitation à deux faisceaux lumineux distincts ;
- la figure 6 illustre l'application d'un procédé selon un mode de réalisation de l'invention à la détection du complexe fluorescent réversiblement photoconvertible « Spinach » ;
- la figure 7 illustre l'application d'un procédé selon un mode de réalisation de l'invention à l'imagerie sélective d'un mélange comprenant le complexe fluorescent réversiblement photoconvertible « Spinach » et un fluorophore interférent à l'intérieur d'un dispositif microfluidique ;
- la figure 8 illustre l'application d'un procédé selon un mode de réalisation de l'invention à l'imagerie sélective d'un mélange comprenant la protéine fluorescente réversiblement photoconvertible « Dronpa-2 » et un fluorophore interférent à l'intérieur d'un dispositif microfluidique ;
- la figure 9 illustre l'application d'un procédé selon un mode de réalisation de l'invention à l'imagerie sélective de matériel biologique exprimant la protéine fluorescente réversiblement convertible « Dronpa-3 » ;
- la figure 10 illustre l'application d'un procédé selon un mode de réalisation de l'invention utilisant deux faisceaux d'excitation à l'imagerie sélective d'un mélange comprenant la protéine fluorescente réversiblement photoconvertible « Dronpa-2 » et un fluorophore interférent à l'intérieur d'un dispositif microfluidique ;

- la figure 11 illustre schématiquement l'appareil expérimental utilisé pour obtenir les figures 7;
- la figure 12 illustre schématiquement un appareil pouvant être utilisé dans des applications de télédétection selon un mode de réalisation de l'invention ; et
- la figure 13 illustre schématiquement l'appareil expérimental utilisé pour obtenir la figure 10;
- la figure 14 illustre schématiquement l'appareil expérimental utilisé pour obtenir la figure 8.

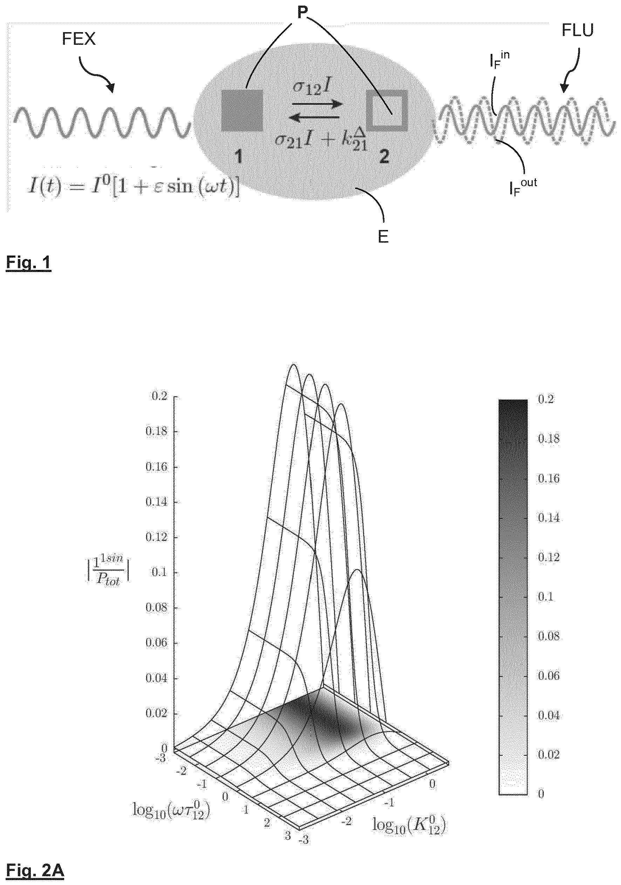

Comme illustré sur la figure 1, un procédé de détection selon l'invention comprend l'éclairage d'un échantillon E, contenant une espèce fluorescente réversiblement photoconvertible **P**, par un faisceau lumineux d'excitation FEX modulé de manière périodique. Dans l'exemple non limitatif de la figure 1 on considère le cas d'un faisceau d'excitation modulé sinusoïdalement à une fréquence angulaire $\omega$, dont l'intensité est donnée par

$$I(t)=I_0[1+\varepsilon \sin (\omega t)]$$

où $I^0$ est l'intensité moyenne et $\varepsilon \leq 1$ est l'amplitude de modulation. Plus généralement on pourra moduler l'intensité du faisceau d'excitation avec toute fonction périodique, dont la fréquence est indiquée par $f=1/T$ (T étant la période ; dans le cas d'une modulation sinusoïdale $f=\omega/2\pi$). L'utilisation d'une modulation par une fonction en créneau (onde rectangulaire, de rapport cyclique quelconque) est particulièrement avantageuse en raison de sa simplicité de mise en oeuvre. La modulation peut également être obtenue en déplaçant périodiquement un faisceau d'éclairage, par exemple dans le cadre d'une observation en microscopie à balayage ; en effet, si on considère une région localisée d'un échantillon (par exemple une chambre ou un conduit d'un dispositif microfluidique), un déplacement périodique d'un faisceau d'éclairage modifie l'illumination de façon analogue à une modulation périodique de l'intensité dudit faisceau.

Le faisceau d'excitation FEX est de préférence sensiblement monochromatique, c'est-à-dire que son spectre présente un seul maximum d'intensité, et/ou une largeur spectrale non supérieure à 50 nm.

L'espèce fluorescente réversiblement photoconvertible présente deux états différents échangeables sous l'action de la lumière. Il peut s'agir d'une espèce fluorescente photochrome, ou de tout autre système dont le comportement dynamique peut se réduire à un échange entre deux états sous l'action de la lumière ; ces états peuvent correspondre à des configurations stéréochimiques différentes d'une molécule, à un état lié/non lié d'un complexe, etc. Sur la figure 1, le premier état - thermodynamiquement plus stable - est indiqué par **1** et représenté par un carré plein ; le second état - thermodynamiquement moins stable - est indiqué par **2** et représenté par un carré creux. Ces deux états possèdent des brillances différentes. Dans un souci de simplicité, et à titre d'exemple non limitatif, on peut considérer que seul l'état **1** est fluorescent de manière significative.

La longueur d'onde du faisceau d'excitation, $\lambda$, permet à la fois l'excitation de l'émission de fluorescence des états **1** et **2,** et également la conversion de l'état **1** vers l'état **2** et vice-versa. Ainsi, en cas d'éclairage avec une intensité constante I :

- l'état **1** est converti en l'état **2** avec une constante cinétique $\sigma_{12}$I, $\sigma_{12}$ étant la section efficace de photoconversion de l'état **1** en l'état **2** ;
- l'état **2** est converti en l'état **1** avec une constante cinétique $\sigma_{21}I+k^{\Delta}_{21}$, $\sigma_{21}$ étant la section efficace de photoconversion de l'état **2** en l'état **1** et $k^{\Delta}_{21}$ étant la constante cinétique de relaxation thermique de l'état **2** vers l'état **1.**

L'échantillon - et plus précisément l'espèce **P** qu'il contient - éclairé par le faisceau d'excitation modulé FEX, émet un rayonnement de fluorescence FLU dont l'intensité est, elle-aussi, modulée et peut être décomposée en :

- une composante en phase avec le faisceau d'excitation, indiquée sur la figure par $I_F^{in}$ ; et
- une composante en quadrature avec le faisceau d'excitation, indiquée sur la figure par $I_F^{out}$.

Les présents inventeurs se sont rendus compte qu'il est possible de choisir l'intensité moyenne $I^0$ du faisceau d'éclairage et sa fréquence de modulation f de manière à maximiser l'amplitude de la composante en quadrature du rayonnement de fluorescence. Les valeurs optimales des paramètres $I^0$ et f qui maximisent cette amplitude dépendent des paramètres $\sigma_{12}$, $\sigma_{21}$, $k^{\Delta}_{21}$ et de l'espèce fluorescente réversiblement photoconvertible considérée. Ainsi, un faisceau d'excitation optimisant l'amplitude de la composante en quadrature de l'émission d'une espèce fluorescente réversiblement photoconvertible cible n'optimisera pas celle d'autres espèces fluorescentes (réversiblement photoconvertibles ou non) pouvant être présentes dans l'échantillon. On obtient ainsi une détection sélective par contraste dynamique.

Une caractéristique avantageuse de l'invention est que les valeurs optimales des paramètres $I^0$ et f peuvent être calculées à partir des propriétés dynamiques de l'espèce à détecter, et plus particulièrement de $\sigma_{12,}$ $\sigma_{21}$ et $k^{\Delta}_{21}$. Il

n'est donc pas nécessaire d'avoir recours à une optimisation itérative, par essais, comme dans certaines techniques de l'art antérieur. Par ailleurs, les valeurs optimales de $I^0$ et de f peuvent être déterminées de manière analytique.

Un autre avantage de l'invention est que la détection sélective peut être quantitative. Autrement dit, un étalonnage permet de déterminer la concentration de l'espèce fluorescente réversiblement photoconvertible cible à partir de l'amplitude de la composante en quadrature de l'intensité de fluorescente.

L'extraction de l'amplitude de la composante en quadrature de l'émission fluorescente ne pose pas de difficulté particulière. Elle peut se faire, par exemple, par détection synchrone (« lock-in » en anglais) ou par analyse de la transformée de Fourier de l'intensité de fluorescence. De même, la modulation du faisceau d'excitation peut être obtenue par des méthodes connues, par exemple la modulation directe d'une source de rayonnement ou l'utilisation d'un modulateur de lumière, électro-optique ou mécanique.

Contrairement à des techniques connues de l'art antérieur, il n'est pas nécessaire de prévoir une pluralité de faisceaux d'excitation. Un seul faisceau d'éclairage - ou une pluralité de faisceaux de même longueur d'onde et même modulation, voire même un éclairage monochromatique diffus - peut être utilisé.

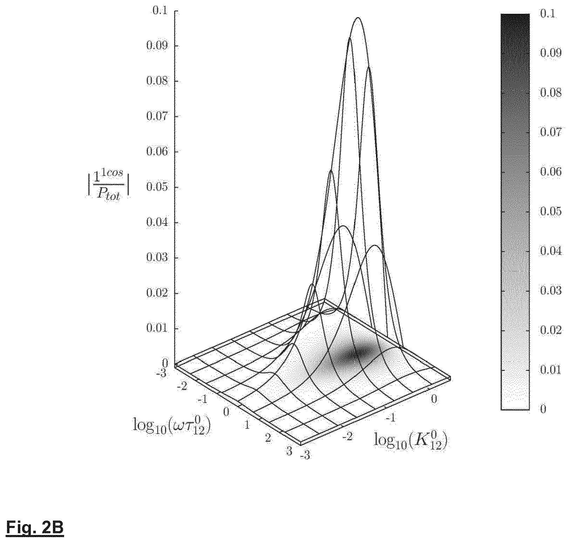

La théorie à la base de l'invention sera maintenant exposée plus en détail à l'aide des figures 2A et 2B.

En l'absence d'éclairage, l'espèce fluorescente photoconvertible **P** existe presque exclusivement dans son état le plus stable, **1.** Lorsque le système est éclairé avec une intensité $I^0$ on observe une photoconversion de l'état **1** vers l'état **2** caractérisée par une constante de vitesse :

$$k_{12}(t) \quad = \quad k_{12}^0 = \sigma_{12} I^0 \tag{1}$$

et une conversion - à la fois thermique et photo-induite - de l'état **2** vers l'état **1** caractérisée par une constante de vitesse :

$$k_{21}(t) \quad = \quad k_{21}^0 = \sigma_{21} I^0 + k_{21}^\Delta \tag{2}$$

Après une période pré-stationnaire dont la durée est définie par le temps de relaxation $\tau_{12}^0 = 1/\left(k_{12}^0 + k_{21}^0\right)$, le système atteint un régime photo-stationnaire, caractérisé par la constante de photoisomérisation apparente

$$K_{12}^0 = k_{12}^0 / k_{21}^0 \tag{3}$$

Dans ce régime, les concentrations des états **1** et **2,** notées $1^0$ et $2^0$ respectivement, sont données par :

$$1^0 \quad = \quad P_{tot} - 2^0 = \frac{1}{1 + K_{12}^0} P_{tot} \tag{4}$$

où $P_{tot}$ est la concentration totale de l'espèce **P.** L'intensité de l'émission de fluorescence $I_F(t)$ est donnée par :

$$I_F(t) \quad = \quad (Q_1 1 + Q_2 2) I(t) = I_F^0 = \left(Q_1 1^0 + Q_2 2^0\right) I^0 \tag{5}$$

où $Q_1$ et $Q_2$ sont les brillances moléculaires des états **1** et **2** respectivement, avec $Q_1 \neq Q_2$.

On considère maintenant le cas d'un éclairage modulé sinusoïdalement à la fréquence angulaire $\omega$ et avec une amplitude de modulation $\varepsilon$ :

$$I(t) = I_0[1 + \varepsilon \sin (\omega t)] \tag{6}$$

Si $\varepsilon \ll 1$ on trouve, au premier ordre :

$$k_{12}(t) = \sigma_{12}I^0\left[1 + \varepsilon \sin\left(\omega t\right)\right]$$
$$k_{21}(t) = \sigma_{21}I^0\left[1 + \varepsilon \sin\left(\omega t\right)\right] + k_{21}^\Delta \tag{7}$$

Il convient de noter que cette hypothèse $\varepsilon \ll 1$ n'est nullement essentielle à la mise en oeuvre de l'invention, mais simplifie le développement analytique.

En d'autres termes, l'invention peut être mise en oeuvre avec une modulation de « forte » amplitude ($\varepsilon$ proche de 1) en conservant les valeurs des paramètres I⁰ et $\omega$ fournies ci-dessous qui optimisent alors l'amplitude du terme d'ordre 1 en quadrature de la modulation des concentrations en **1** et **2**.

Au-delà du temps de relaxation $\tau_{12}^0$, le système entre dans un régime forcé permanent, dans lequel les concentrations des deux états **i** (**i=1** ou **2**) valent :

$$i(t) = i^0 + \varepsilon\left[i^{1sin}\sin\left(\omega t\right) + i^{1cos}\cos\left(\omega t\right)\right] \tag{8}$$

où i⁰ est la concentration de **i** à l'état stationnaire associé au flux photonique I⁰ tandis que $\varepsilon i^{1sin}\sin(\omega t)$ et $\varepsilon i^{1cos}\cos(\omega t)$ sont les termes en phase et en quadrature oscillant à la fréquence angulaire $\omega$. On peut montrer que :

$$1^{1sin} = -2^{1sin} = -\rho_{12}^0\tau_{12}^0 p_{21}^\Delta \frac{1}{1 + \left(\omega\tau_{12}^0\right)^2} \tag{9}$$

et

$$1^{1cos} = -2^{1cos} = \rho_{12}^0\tau_{12}^0 p_{21}^\Delta \frac{\omega\tau_{12}^0}{1 + \left(\omega\tau_{12}^0\right)^2} \tag{10}$$

où $\rho_{12}^0 = k_{12}^0 1^0 = k_{21}^0 2^0$ et $p_{21}^\Delta = k_{21}^\Delta/(\sigma_{21}I^0 + k_{21}^\Delta)$.

L'intensité de l'émission de fluorescence I_F(t) devient alors :

$$I_F(t) = I_F^0 + I_F^{in}sin(\omega t) + I_F^{out}cos(\omega t) \tag{11}$$

avec :

$$I_F^{in} = \epsilon[(Q_1 1^0 + Q_2 2^0) + (Q_1 - Q_2)1^{1sin}]I^0 \tag{12}$$

$$I_F^{out} = \epsilon(Q_1 - Q_2)1^{1cos}I^0 \tag{13}$$

En divisant par la valeur moyenne $I_F^0$ on obtient l'émission de fluorescence normalisée :

$$I_{F,norm}(t) \;=\; \frac{I_F(t) - I_F^0}{I_F^0} = \left(1 + \alpha^{\mathrm{in}}\right)\varepsilon\sin\left(\omega t\right) + \alpha^{\mathrm{out}}\varepsilon\cos\left(\omega t\right)$$

$$(14)$$

où $\alpha^{\mathrm{in}}$ et $\alpha^{\mathrm{out}}$ sont les amplitudes des composantes en phase et en quadrature, respectivement, définies par: $\alpha^{\mathrm{in}} = (Q_1 - Q_2)\, 1^{1sin}/A^0$ et $\alpha^{\mathrm{out}} = (Q_1 - Q_2)\, 1^{1cos}/A^0$ avec $A^0 = Q_1 1^0 + Q_2 2^0$.

Pour une intensité d'éclairage moyenne fixée, $I^0$, le triplet de paramètres $\left(\sigma_{12}, \sigma_{21}, k_{21}^{\Delta}\right)$ caractérisant le fluorophore réversiblement photoconvertible **P** donne deux ensembles de paramètres dynamiques équivalents : $\left(k_{12}^0, k_{21}^0\right)$ et $\left(K_{12}^0, \tau_{12}^0\right)$ ; ce second ensemble est sélectionné pour caractériser la dynamique du système. Les termes $1^{1sin}$ et $1^{1cos}$, dont dépendent les composantes en phase et en quadrature de l'intensité de fluorescence, peuvent alors être exprimés par :

$$1^{1sin} \;=\; -p_{21}^{\Delta}\frac{K_{12}^0}{\left(1 + K_{12}^0\right)^2}\frac{1}{1 + \left(\omega\tau_{12}^0\right)^2}P_{tot}$$

$$(15)$$

et

$$1^{1cos} \;=\; p_{21}^{\Delta}\frac{K_{12}^0}{\left(1 + K_{12}^0\right)^2}\frac{\omega\tau_{12}^0}{1 + \left(\omega\tau_{12}^0\right)^2}P_{tot}$$

$$(16)$$

Les figures 2A et 2B illustrent, respectivement, la dépendance de l'amplitude normalisée en phase $|1^{1sin}/P_{tot}|$ et de l'amplitude normalisée en quadrature $|1^{1cos}/P_{tot}|$ en fonction de $K_{12}^0$ et $\omega\tau_{12}^0$ (exprimés en unités logarithmiques). On peut remarquer que $1^{1cos}$ - et donc la composante en quadrature $I_F^{\mathrm{out}}$ de l'intensité d'émission fluorescente - présente un maximum bien défini, ce qui n'est pas le cas de $1^{1sin}$. Ce maximum est centré sur le point :

$$K_{12}^0 \;=\; \frac{\sigma_{12}}{\sigma_{12} + 2\sigma_{21}}$$

$$(17)$$

$$\omega\tau_{12}^0 \;=\; 1$$

$$(18)$$

Il est possible de choisir les paramètres de contrôle ($I^0$, $\omega$) de manière à satisfaire ces conditions. La valeur de $1^{1cos}$ - et de $I_F^{\mathrm{out}}$ - est donc maximale lorsque :

$$I^0 = \frac{k_{21}^{\Delta}}{\sigma_{12} + \sigma_{21}}$$

$$(19)$$

et

$$\omega = 2k_{21}^{\Delta} \qquad (20)$$

ou, de manière équivalente :

$$f = k_{21}^{\Delta}/\pi. \qquad (21)$$

Physiquement, la condition (19) sur $I^0$ revient à égaliser les constantes cinétiques des réactions thermiques et photo-induites, et la condition (20-21) sur $\omega$ ou f à accorder la fréquence de modulation sur le temps de relaxation $\tau_{12}^0$ pour vérifier la condition de résonance (18).

L'utilisation d'un signal de modulation de faible amplitude est propice à la dérivation d'expressions analytiques simples. En revanche, elle ne permet de générer que de faibles variations du signal de l'espèce P à cause des faibles modulations, ce qui représente un inconvénient pour une extraction fiable d'une réponse du premier ordre en quadrature de phase. On peut analyser la réponse d'une espèce photoconvertible P dans le cas d'une modulation de large amplitude a pour pallier cet inconvénient. On peut d'abord considérer, comme une extension du cas précédent, une modulation sinusoïdale de large amplitude $\alpha$ telle que :

$$I(t) = I^0 \left[1 + \alpha \sin(\omega t)\right] \qquad (22)$$

Pour calculer la dépendance temporelle des concentrations en espèces fluorescentes réversiblement photoconvertibles, on peut d'abord considérer que, dans un cas plus général, l'intensité lumineuse peut être écrite sous la forme :

$$I(t) = I^0 \left[1 + \alpha h(\omega t)\right] \qquad (24)$$

où $h(\omega t)$ désigne une fonction périodique de pulsation fondamentale $\omega$. On peut utiliser l'équation 24 pour développer les constantes de vitesse des équations 1 et 2. En écrivant l'expression des concentrations de la manière suivante :

$$2 = 2^0 + \alpha f(\omega t) \qquad (25)$$

$$1 = 1^0 - \alpha f(\omega t) \qquad (26)$$

le système d'équations différentielles régissant l'évolution temporelle des concentrations **1** et **2** devient :

$$\frac{df(\theta x)}{dx} = -f(\theta x) + \left[a - bf(\theta x)\right] h(\theta x) \qquad (27)$$

où :

$$x = \frac{t}{\tau_{12}^0} \qquad (28)$$

$$a = \rho_{12}^0 p_{21}^\Delta \tau_{12}^0 \qquad (29)$$

$$b = \alpha(\sigma_{12} + \sigma_{21}) I^0 \tau_{12}^0 \qquad (30)$$

$$\theta = \omega \tau_{12}^0 \qquad (31)$$

Après le temps de relaxation $\tau_{12}^0$, un régime permanent, dans lequel $f(\theta x)$ est une fonction continue périodique, s'établit. Contrairement au cas d'une modulation sinusoïdale de faible amplitude, on ne peut pas restreindre l'analyse de $f(\theta x)$ au premier ordre. La fonction $f(\theta x)$ doit être développée en série de Fourier :

$$f(\theta x) = a_0 + \sum_{n=1}^{+\infty} [a_n \cos(n\theta x) + b_n \sin(n\theta x)] \qquad (32)$$

où $a_n$ et $b_n$ désignent les amplitudes des n-ièmes composantes de la série de Fourier. Les termes $a_n$ et $b_n$ peuvent être extraits de l'équation 27 par identification des amplitudes du même ordre. On peut ainsi obtenir l'expression des concentrations **1** et **2** :

$$2 = 2^0 + \alpha \left\{ a_0 + \sum_{n=1}^{+\infty} [a_n \cos(n\theta x) + b_n \sin(n\theta x)] \right\} \qquad (33)$$

$$1 = 1^0 - \alpha \left\{ a_0 + \sum_{n=1}^{+\infty} [a_n \cos(n\theta x) + b_n \sin(n\theta x)] \right\}. \qquad (34)$$

En conséquence, en régime permanent, une modulation de large amplitude de l'illumination entraîne la modulation des concentrations **1** et **2** sur une infinité de pulsations. Les équations 33 et 34 peuvent être transformées pour faire apparaître les amplitudes $i^{n,in}$ et $i^{n,out}$ des termes en phase ou en quadrature de phase oscillant à la pulsation $n\omega$. La concentration de **i** peut être écrite :

$$i = i^0 + \alpha \sum_{n=1}^{+\infty} \left[ i^{n,in} \sin(n\omega t) + i^{n,out} \cos(n\omega t) \right]. \qquad (35)$$

Les termes $i^0$, $i^{n,in}$ et $i^{n,out}$ sont proportionnels à $P_{tot}$. En effet l'équation 27 peut être transformée en

$$\frac{df(\theta x)}{dx} + f(\theta x)[1 + bh(\theta x)] = ah(\theta x). \qquad (36)$$

Ni $b$ ni $h(\theta x)$ ne dépendent de $P_{tot}$ (voir équations 22, 23, 28-31). En revanche, $a$ est proportionnel à $P_{tot}$ (voir équations 4 et 29). La dérivation étant linéaire, l'équation 36 implique que $f(\theta x)$ est proportionnel à $P_{tot}$. Le système d'équation donnant accès aux $a_n$ et $b_n$ est linéaire. On peut donc déduire que toutes les amplitudes $a_n$ et $b_n$ sont individuellement proportionnelles à $P_{tot}$. Finalement, on utilise les équations 33 à 35 pour déduire que les $i^0$, $i^{n,in}$ et $i^{n,out}$ sont proportionnelles à $P_{tot}$. On a montré précédemment que dans le cas d'une modulation sinusoïdale de la lumière de petite amplitude, $2^{1,out}$ est optimal quand les conditions de résonnance des équations 19 et 21 sont remplies. En l'absence d'une expression analytique pour $2^{1,out}$, de telles conclusions ne peuvent pas être tirées dans le cas d'une modulation de la lumière périodique de forte amplitude. Leur pertinence a été évaluée au moyen de calculs numériques.

Dans un exemple de réalisation de l'invention, le cas des modulations sinusoïdales de large amplitude a été analysé. Plus particulièrement, la dépendance $1^{1,out} = -2^{1,out} = -a_1$ a été analysée sur les paramètres de contrôle $\omega$ et $I^0$. A cet effet, les $2n + 1$ paramètres inconnus $(a_0, ..., a_n, b_n)$ sont retrouvés par troncature du développement en série de Fourier (équation 32) pour des ordres de n croissants.

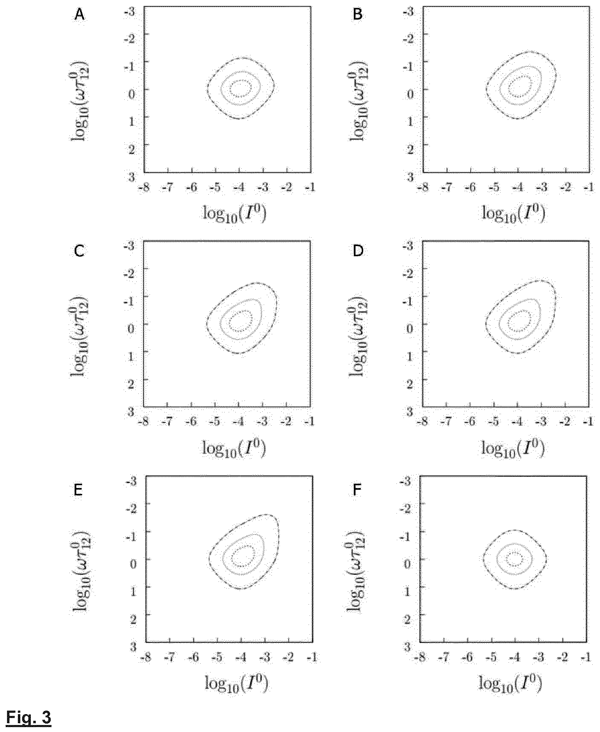

La figure 3 présente la dépendance de l'amplitude normalisée $\left| 1_{norm}^{1,out} \right| = \left| 1^{1,out} / P_{tot} \right|$, sur le flux lumineux $I^0$ (en ein.s$^{-1}$.m$^{-2}$) et la pulsation adimensionnée $\omega \tau_{12}^0$ quand $\alpha = 1$. Le calcul numérique est réalisé sur la troncature de la série de Fourier $f(\theta x)$ à l'ordre 1,2,3,4 et 5 respectivement pour les panneaux A,B,C,D et E. Le panneau F présente la dépendance de l'amplitude normalisée sur les mêmes variables, observée dans un régime de modulation de faible amplitude. Les marqueurs correspondent aux courbes isodensité (0,01 pour les points-tirets, 0,03 pour les points et 0,05 pour les tirets). La troncature du développement en série de Fourier de la fonction $f(\theta x)$ à l'ordre 5 ($n = 5$) est suffisante pour observer une convergence : la dépendance de $\left| 1_{norm}^{1,out} \right|$ sur $I^0$ et $\omega$ n'évolue plus significativement au-delà de $n = 5$. $\left| 1_{norm}^{1,out} \right|$ présente un optimum dans l'espace ($I^0, \omega$), pour lequel la position et l'amplitude sont très proches de celles observées dans le cas d'une modulation sinusoïdale de faible amplitude. L'erreur faite en prenant l'expression analytique, valide seulement pour la modulation de faible amplitude, est inférieure à 20%, quelque soit l'amplitude de $\alpha$ utilisée. Une telle erreur est de l'ordre de l'erreur expérimentale en fixant l'intensité lumineuse moyenne et la pulsation à leurs valeurs de résonance, $I^{0,R}$ et $\omega^R$.

Si, au lieu de considérer une seule espèce fluorescente réversiblement photoconvertible on s'intéresse à un échantillon contenant une pluralité de telles espèces, présentant des paramètres dynamiques différents mais des bandes d'absorption/émission superposées ou proches (inclues dans la largeur spectrale de la lumière d'éclairage), l'intensité en quadrature présentera une pluralité de maxima locaux, un pour chaque dite espèce.

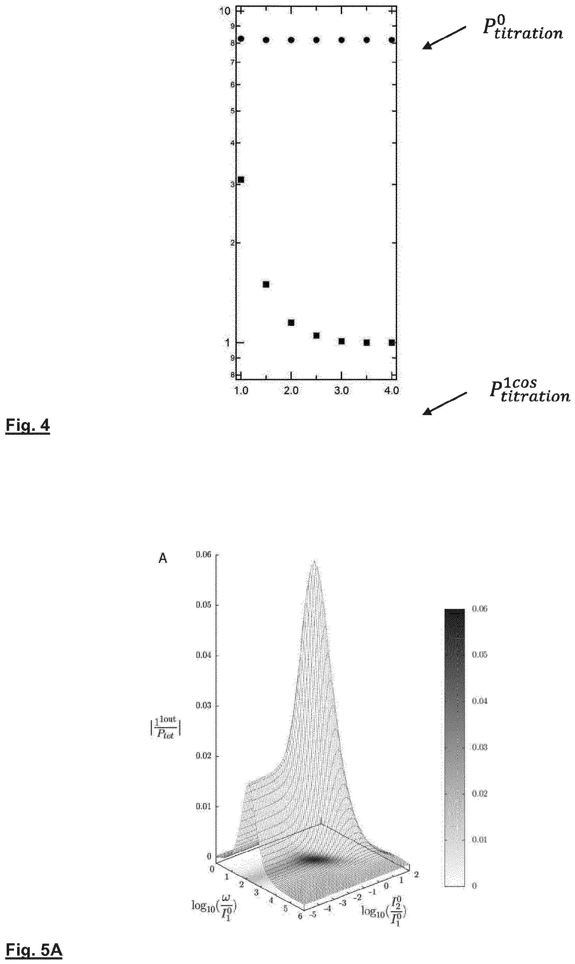

La figure 4 permet de mettre en évidence la sélectivité de la détection selon l'invention, ainsi que son caractère quantitatif.

Dans cette figure, on considère le cas d'un échantillon contenant neuf espèces fluorescentes réversiblement photoconvertibles, ayant une même concentration, des longueurs d'onde d'absorption et d'émission identiques et un état **1** de même brillance (l'état **2** est considéré comme non fluorescent, sans perte de généralité) : une espèce cible et huit espèces interférentes. Dans l'espace dont les dimensions sont : $log_{10} \dfrac{k_{21,X}^{\Delta}}{k_{21}^{\Delta}}$, $log_{10} \dfrac{\sigma_{12,X}}{\sigma_{12}}$, $log_{10} \dfrac{\sigma_{21,X}}{\sigma_{21}}$ (« X » identifie les espèces interférentes, les paramètres sans cet indice se réfèrent à l'espèce cible), les espèces interférentes forment un cube d'arête « n », avec l'espèce cible au centre. On considère sept cas différents, correspondant aux valeurs suivantes de « n » : 1 ; 1,5 2 ; 2,5 ; 3 ; 3,5 ; 4.

Si on essaie de titrer l'espèce cible par détection de fluorescence en utilisant un éclairage d'intensité constante, la mesure est très fortement perturbée par les espèces interférentes. On trouve, plus exactement, et indépendamment de la valeur de « n » :

$$P_{titration}^0 = P_{tot} + \sum_X \frac{(1_X^0 / X_{tot})}{(1^0 / P_{tot})} X_{tot} \tag{37}$$

où $P_{titration}^0$ est la concentration mesurée de la cible, extraite de la quantification de l'intensité moyenne de fluorescence, $P_{tot}$ est sa concentration réelle, $X_{tot}$ la concentration totale de l'espèce interférente X, $1^0$ et $1_X^0$ les concentrations stationnaires sous illumination d'intensité constante $I^0$ de la cible et des espèces interférentes X, respectivement. Comme le montre le graphique de la figure 4, la concentration est surestimée d'un facteur supérieur à 8.

Si par contre on utilise un éclairage modulé et une détection en quadrature de la fluorescence, on obtient :

$$P_{titration}^{1cos} = P_{tot} + \sum_{X} \frac{(1_X^{1cos}/X_{tot})}{(1^{1cos}/X_{tot})} X_{tot} \qquad (38)$$

où $P_{titration}^{1cos}$ est la concentration mesurée de la cible, extraite de l'analyse de la réponse en quadrature, et $1^{1cos}$ et $1_X^{1cos}$ sont les amplitudes des composantes en quadrature des concentrations des états **1** de la cible et des espèces interférentes X.

Lorsque la fréquence de modulation et l'intensité de l'éclairage sont optimisées pour la détection de l'espèce cible, $1^{1cos}/P_{tot}$ est très supérieur à $1_X^{1cos}/X_{tot}$. Le graphique de la figure 4 montre que $P_{titration}^{1cos}$ est d'autant plus proche de $P_{tot}$ que le paramètre « n » est élevé. Lorsque les propriétés dynamiques des espèces interférentes sont suffisamment éloignées de celles de l'espèce cible (n≥2 dans l'exemple considéré ici), la surestimation qu'elles provoquent est négligeable. La sélectivité obtenue grâce au procédé de l'invention permet donc une détection quantitative (titrage) même en présence d'espèces interférentes - à conditions que ces dernières présentent des paramètres dynamiques suffisamment différents de ceux de l'espèce cible, ce qui est généralement le cas, d'autant plus que la plupart des fluorophores interférents naturellement présents dans les échantillons, notamment biologiques, ne sont pas réversiblement photoconvertibles.

Les résultats présentés sur la figure 4 ont été obtenus par simulation numérique, en considérant les paramètres suivants : $\sigma_{12}$=20,9 m²·mol⁻¹ ; $\sigma_{21}$=6,8 m²·mol⁻¹ ; $k_{21}^{\Delta}$=2,8·10⁻³ s⁻¹.

En variante, on peut utiliser deux faisceaux d'excitation distincts, à des longueurs d'onde différentes : chacun des deux faisceaux, de spectre électromagnétique et d'intensité lumineuse différente, peut imposer des constantes de vitesse différentes aux réactions liant les deux états de l'espèce P. L'un des désavantages de l'utilisation de deux faisceaux est de compliquer la mise en oeuvre du procédé de détection. En revanche, il permet avantageusement de s'affranchir de la limitation de la vitesse d'acquisition liée à la relaxation thermique de l'état **2** vers l'état **1** dans le cas de l'utilisation d'un seul faisceau lumineux.

Dans cette réalisation particulière de l'invention, l'espèce P est illuminée par une lumière d'intensité $I(t)$, comprenant des composantes $I_1(t)$ et $I_2(t)$, respectivement de longueurs d'onde $\lambda_1$ et $\lambda_2$. Le système à deux états précédemment décrit possède alors les constantes de vitesse suivantes :

$$k_{12}(t) = \sigma_{12,1}I_1(t) + \sigma_{12,2}I_2(t) \qquad (39)$$

$$k_{21}(t) = \sigma_{21,1}I_1(t) + \sigma_{21,2}I_2(t) + k_{21}^{\Delta} \qquad (40)$$

où : $\sigma_{12,1}I_1(t)$, $\sigma_{12,2}I_2(t)$, $\sigma_{21,1}I_1(t)$, $\sigma_{21,2}I_2(t)$ et $k_{21}^{\Delta}$ sont respectivement les contributions photochimiques et thermiques des constantes de vitesse. Dans ce cas, les sections moléculaires efficaces pour la photoisomérisation $\sigma_{12,1}$ et $\sigma_{21,1}$ (à $\lambda_1$), $\sigma_{12,2}$ et $\sigma_{21,2}$ (à $\lambda_2$), et la constante de relaxation thermique $k_{21}^{\Delta}$ définissent complètement le comportement d'une espèce photoconvertible P.

Dans une réalisation particulière de l'invention, on utilise ce système avec une illumination à la longueur d'onde $\lambda_1$, d'intensité périodique sinusoïdale oscillant autour de la valeur moyenne $I_1^0$ à une pulsation $\omega_1$ et avec une petite amplitude d'oscillation $\varepsilon I_1^0$ ($\varepsilon \ll 1$), sur laquelle on superpose une illumination de longueur d'onde $\lambda_2$, avec $\lambda_1 \neq \lambda_2$, d'intensité constante $I_2^0$. On a alors :

$$I(t) = I_1^0 [1 + \varepsilon \sin(\omega t)] + I_2^0. \qquad (41)$$

En introduisant :

$$k_{12,1}^0 \quad = \quad \sigma_{12,1} I_1^0 \tag{42}$$

$$k_{21,1}^0 \quad = \quad \sigma_{21,1} I_1^0 \tag{43}$$

$$k_{12,2}^0 \quad = \quad \sigma_{12,2} I_2^0 \tag{44}$$

$$k_{21,2}^0 \quad = \quad \sigma_{21,2} I_2^0 \tag{45}$$

On écrit :

$$k_{12}(t) \quad = \quad k_{12,1}^0 \left[1 + \varepsilon \sin\left(\omega t\right)\right] + k_{12,2}^0 \tag{46}$$

$$k_{21}(t) \quad = \quad k_{21,1}^0 \left[1 + \varepsilon \sin\left(\omega t\right)\right] + k_{21,2}^0 + k_{21}^\Delta, \tag{47}$$

et le système d'équations différentielles gouvernant l'évolution temporelle des concentrations **1** et **2** est résolu lors d'un développement du premier ordre pour cette perturbation lumineuse. Après le temps de relaxation $\tau_{12}^0$, un régime forcé et permanent s'établit, où la concentration :

$$i = i^0 + \varepsilon i^1 \sin\left(\omega t - \phi_{12}\right) \tag{48}$$

en chaque espèce **i** (**i = 1** ou **2**) oscille autour d'une valeur moyenne $i^0$ (qui correspond à la concentration de **i** en régime permanent associée au flux de photon $I^0$ ; voir équation 4) à la pulsation $\omega$ mais avec un retard de phase de $\phi_{12} = \arctan\left(\omega \tau_{12}^0\right)$. Les amplitudes des modulations de concentration sont données par :

$$2^1 \quad = \quad -1^1 = \frac{\rho_{12}^0 \tau_{12}^0 \Delta_{12}^0}{\sqrt{1 + \left(\omega \tau_{12}^0\right)^2}} \tag{49}$$

où :

$$\rho_{12}^0 \quad = \quad k_{12}^0 1^0 = k_{21}^0 2^0 \tag{50}$$

et :

$$\Delta_{12}^0 \quad = \quad \frac{k_{12,1}^0}{k_{12,1}^0 + k_{12,2}^0} - \frac{k_{21,1}^0}{k_{21,1}^0 + k_{21,2}^0 + k_{21}^\Delta} \tag{51}$$

désigne la vitesse de la réaction en régime permanent et les contributions des processus photochimiques par la lumière modulée, à la transition de **1** à **2** et respectivement de **2** à **1** en illuminant à $I^0$.

Les concentrations i peuvent aussi être écrites :

$$i(t) = i^0 + \varepsilon \left[ i^{1,in} \sin(\omega t) + i^{1,out} \cos(\omega t) \right] \tag{52}$$

où $\varepsilon i^{1,in} \sin(\omega t)$ et $\varepsilon i^{1,out} \cos(\omega t)$ sont les termes oscillants en phase et en quadrature de phase à la pulsation $\omega$. Les amplitudes $i^{1,in}$ et $i^{1,out}$ des termes en phase et en quadrature de phase sont :

$$2^{1,in} = -1^{1,in} = \rho_{12}^0 \tau_{12}^0 \Delta_{12}^0 \frac{1}{1 + \left(\omega \tau_{12}^0\right)^2} = \Delta_{12}^0 \frac{K_{12}^0}{\left(1 + K_{12}^0\right)^2} \frac{1}{1 + \left(\omega \tau_{12}^0\right)^2} P_{tot} \tag{53}$$

$$2^{1,out} = -1^{1,out} = -\rho_{12}^0 \tau_{12}^0 \Delta_{12}^0 \frac{\omega \tau_{12}^0}{1 + \left(\omega \tau_{12}^0\right)^2} = -\Delta_{12}^0 \frac{K_{12}^0}{\left(1 + K_{12}^0\right)^2} \frac{\omega \tau_{12}^0}{1 + \left(\omega \tau_{12}^0\right)^2} P_{tot} \tag{54}$$

dans lesquels $\rho_{12}^0 \tau_{12}^0 = P_{tot} \left[ K_{12}^0 / \left(1 + K_{12}^0\right)^2 \right]$.

Comme l'émission fluorescente provient des contributions des espèces **1** et **2,** le retard de phase dans les concentrations oscillantes a pour conséquence un retard de phase de l'émission fluorescente oscillante. L'émission fluorescente oscillante est alors :

$$I_F(t) = I_F^0 + I_F^{1,in} \sin(\omega t) + I_F^{1,out} \cos(\omega t) \tag{55}$$

où les amplitudes $I_F^{1,in}$ et $I_F^{1,out}$ des termes en phase et en quadrature de phase sont :

$$I_F^{1,in} = \varepsilon \left[ \left( Q_1 1^0 + Q_2 2^0 \right) I_1^0 + \left( Q_1 - Q_2 \right) 1^{1,in} I^0 \right] \tag{56}$$

$$I_F^{1,out} = \varepsilon \left( Q_1 - Q_2 \right) 1^{1,out} I^0. \tag{57}$$

avec $I^0 = I_1^0 + I_2^0$.

La réalisation de l'invention utilisant deux faisceaux d'excitation distincts diffère de la réalisation de l'invention utilisant un seul faisceau d'excitation dans la limite où les échanges entre les états 1 et 2 sont essentiellement dus aux contributions photochimiques lors de l'utilisation de deux faisceaux.

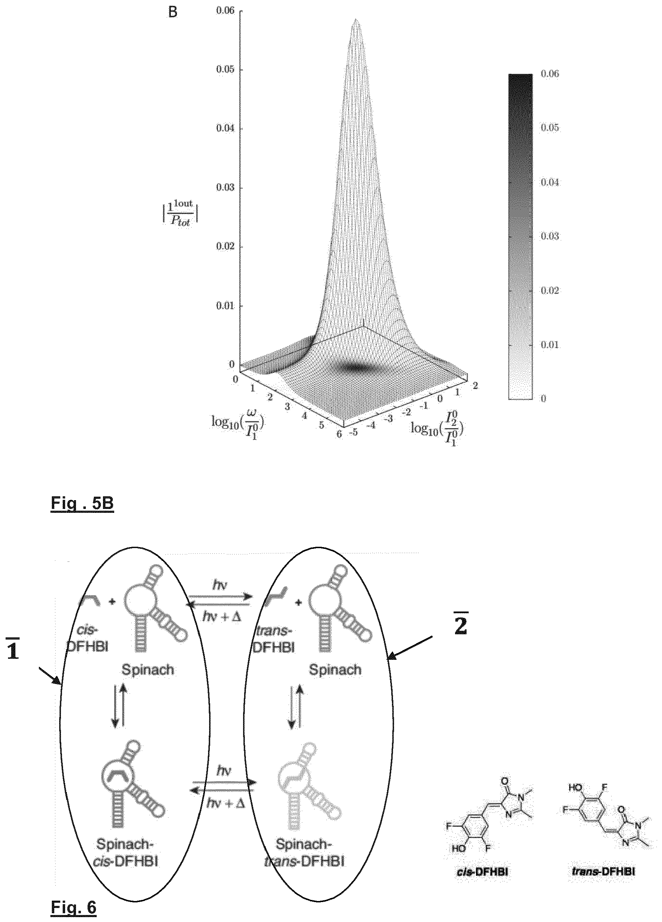

On peut, lors de la réalisation de l'invention utilisant deux faisceaux d'excitation, calculer des paramètres de contrôle pour optimiser la réponse en quadrature de phase. L'analyse est choisie dans la gamme d'intensité $(I_1^0, I_2^0)$ tel que $\sigma_{21,1} I_1^0 + \sigma_{21,2} I_2^0 \gg k_{21}^\Delta$ . Les panneaux A et B de la figure 5 illustrent ces calculs. Dans la figure 5, l'espèce fluorescente P utilisée est la Dronpa-2. Elle est caractérisée par le quintuplet de paramètres ($\sigma_{12,1}$, $\sigma_{21,1}$, $\sigma_{12,2}$, $\sigma_{21,2}$, $k_{12}^\Delta$) tel que $\sigma_{12,1} \gg \sigma_{12,2}$ et $\sigma_{21,2} \gg \sigma_{21,1}$. Son amplitude normalisée en quadrature de phase $\left| \Delta 1_{norm}^{out} \right| = \left| 1^{1,out} / \varepsilon P_{tot} \right|$ est présentée en figure 5 en fonction des paramètres de contrôle $I_2^0 / I_1^0$ et $\omega / I_1^0$. $\left| \Delta 1_{norm}^{out} \right|$ montre un optimum singulier quand les deux conditions de résonnance suivantes sont remplies :

$$\frac{I_2^0}{I_1^0} = \frac{\sigma_{12,1} + \sigma_{21,1}}{\sigma_{12,2} + \sigma_{21,2}} \qquad (58)$$

$$\frac{f'}{I_1^0} = \left(\sigma_{12,1} + \sigma_{21,1}\right)/\pi \qquad (59)$$

L'optimisation de $1^{1,out}$ provient de l'optimisation indépendante des termes $\rho_{12}^0 \tau_{12}^0 \Delta_{12}^0$ et $\dfrac{\omega \tau_{12}^0}{1 + \left(\omega \tau_{12}^0\right)^2}$ dans l'équation 54. $\rho_{12}^0 \tau_{12}^0 \Delta_{12}^0$ mesure l'écart de composition $\Delta 2^0$ du régime permanent $2^0$ après un saut d'intensité lumineuse d'amplitude $\Delta I_1^0 = \varepsilon I_1^0$. Cette composante est maximisée quand les réactions photochimiques induites par les deux sources de lumière ont lieu à la même vitesse. Le second terme optimisé, $\omega \tau_{12}^0 / \left[1 + \left(\omega \tau_{12}^0\right)^2\right]$ est maximisé en ajustant la pulsation $\omega$ au temps de relaxation d'échange $\tau_{12}^0$ tel que $\omega \tau_{12}^0 = 1$. Quand $\omega \gg 1/\tau_{12}^0$, l'échange est lent comparé aux variations de lumière, et le couple {**1**,**2**} n'a pas assez de temps pour répondre. Les termes $i^{1,in}$ et $i^{1,out}$ disparaissent alors. Inversement, quand $\omega \ll 1/\tau_{12}^0$ $i^{1,out}$ s'annule, les concentrations **1** et **2** oscillent alors en phase avec la modulation de lumière.

Le panneau A de la figure 5 présente l'amplitude normalisée des oscillations en quadrature de phase $\left|\Delta 1_{norm}^{out}\right| = \left|1^{1,out}/\varepsilon P_{tot}\right|$ dans le cas où $I_1^0 = 10 \dfrac{k_{21}^{\Delta}}{\sigma_{12,1} + \sigma_{21,1}}$. Le panneau B de la figure 5 présente l'amplitude normalisée des oscillations en quadrature de phase $\left|\Delta 1_{norm}^{out}\right| = \left|1^{1,out}/\varepsilon P_{tot}\right|$ dans le cas où $I_1^0 = 100 \dfrac{k_{21}^{\Delta}}{\sigma_{12,1} + \sigma_{21,1}}$.

Dans une réalisation particulière de l'invention, on peut utiliser deux faisceaux de lumière distincts dont l'intensité d'un des faisceaux de lumière est modulée avec une large amplitude par rapport à la valeur moyenne de son intensité, comme dans le cas de l'éclairage avec une source lumineuse.

L'invention a également été validée expérimentalement.

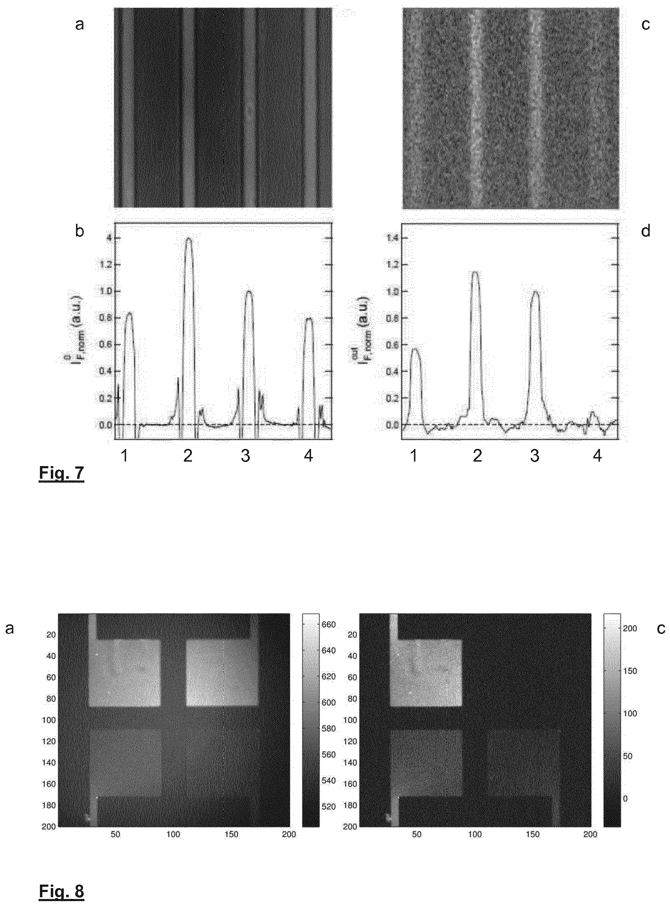

Une première validation, illustrée par la figure 7 utilise, en tant qu'espèce fluorescente réversiblement photoconvertible, le système « Spinach - DFHBI », illustré par la figure 6.

« Spinach » est un aptamère capable de complexer des petites molécules. DFHBI est un fluorogène pouvant être complexé par « Spinach » et existant sous forme de deux isomères, *cis* et *trans.* Le système existe donc en quatre états :

- cis-DFHBI libre, indiqué par **1**$_{free}$ ;
- trans-DFHBI libre, indiqué par **2**$_{free}$ ;
- cis-DFHBI complexé, indiqué par **1**$_{bound}$ ;
- trans-DFHBI complexé, indiqué par **2**$_{bound}$.

Les états liés (complexés) sont fluorescents, contrairement aux états libres, et en particulier l'état **1**$_{bound}$ est à la fois plus stable et plus brillant que l'état **2**$_{bound}$. La réaction d'isomérisation trans - cis (2 →1) se produit à la fois thermiquement (symbole « $\Delta$ » sur la figure 6) et sous l'effet d'un éclairage (symbole « h$\nu$ »), tandis que la réaction cis-trans (1→2) est exclusivement photoinduite (symbole « hv »).

En régime de faible éclairage, les réactions de photoisomérisation sont lentes par rapport aux réactions de complexation/décomplexation. Par conséquent, les paires d'états (**1**$_{free}$ ; **1**$_{bound}$) et (**2**$_{free}$ ; **2**$_{bound}$) peuvent être considérées comme des états virtuels $\overline{\mathbf{1}}$ et $\overline{\mathbf{2}}$. Ainsi, le système « Spinach - DFHBI » peut être considéré comme une espèce fluorescente réversiblement photoconvertible, et la théorie exposée plus haut s'applique.

Un dispositif microfluidique comprenant quatre canaux (profondeur 200 $\mu$m, largeur 50 $\mu$m) a été utilisé comme échantillon ; les canaux 1 à 4 du dispositif ont été remplis avec les solutions suivantes :

1 : (Spinach 250 nM, DFHBI 2,5 $\mu$M) + 75 nM Fluorescéine;

2 : (Spinach 500 nM, DFHBI 5 $\mu$M) + 50 nM Fluorescéine ;

3 : (Spinach 500 nM, DFHBI 5 $\mu$M) seul ;

4 : Fluorescéine 100 nM seule.

Le système (Spinach - DFHBI) constitue l'espèce cible et la Fluorescéine un fluorophore interférent.

Le panneau « a » de la figure 7 montre une image de fluorescence de ce dispositif microfluidique acquise, au moyen d'un microscope, en présence d'un éclairage constant ; le panneau « b » montre l'intensité de fluorescence normalisée correspondante, $I^0_{F,norm}$, intégrée sur la longueur des canaux imagés. Les panneaux « c » et « d » correspondent au cas d'un éclairage modulé et d'une détection en quadrature, dans des conditions optimales pour la détection de l'espèce cible.

On peut observer sur le panneau « d » que les intensités de fluorescence $I^{out}_{F,norm}$, mesurées conformément à l'invention, sont sensiblement proportionnelles aux concentrations de (Spinach - DFHBI) : deux fois plus importantes dans les canaux 2 et 3 que dans le canal 1 et zéro dans le canal 4. Au contraire, les mesures effectuées en éclairage constant (panneau « b ») sont considérablement perturbées par la présence de Fluorescéine (intensité dans le canal 4 sensiblement égale à celle dans le canal 1, alors que l'espèce cible y est absente).

Une seconde validation, illustrée par la figure 8 utilise, en tant qu'espèce fluorescente réversiblement photoconvertible, la protéine «Dronpa-2».

Un dispositif microfluidique comprenant quatre chambres carrées (longueur x largeur x épaisseur = 400 x 400 x 20 $\mu$m$^3$) a été utilisé comme échantillon ; les chambres 1 à 4 du dispositif ont été remplies avec les solutions suivantes :

1 : (Dronpa-2 20 $\mu$M, BSA 100 $\mu$M) (en haut à gauche) ;

2 : Fluorescéine 1 $\mu$M seule (en haut à droite) ;

3 : (Dronpa-2 10 $\mu$M, BSA 100 $\mu$M) (en bas à gauche) ;

4 : (Dronpa-2 5 $\mu$M, BSA 100 $\mu$M) (en bas à droite);

Le système Dronpa-2 constitue l'espèce cible et la Fluorescéine un fluorophore interférent.

Le panneau « a » de la figure 8 montre une image de fluorescence de ce dispositif microfluidique acquise, au moyen d'un microscope, en présence d'un éclairage constant. Le panneau « b » correspond au cas d'un éclairage modulé et d'une détection en quadrature, dans des conditions optimales pour la détection de l'espèce cible.

On peut observer sur le panneau « b » que les intensités de fluorescence $I^{out}_{F,norm}$, mesurées conformément à l'invention, sont sensiblement proportionnelles aux concentrations de Dronpa-2 : un rapport 4 et 2 pour les chambres 1 et 3 comparé à la chambre 4. On observe également que l'intensité de fluorescence $I^{out}_{F,norm}$ est proche de zéro dans le carré contenant uniquement la Fluorescéine.

Bien entendu, d'autres espèces fluorescentes réversiblement photoconvertibles que celles mentionnées ci-dessus peuvent être utilisées pour mettre en oeuvre l'invention.

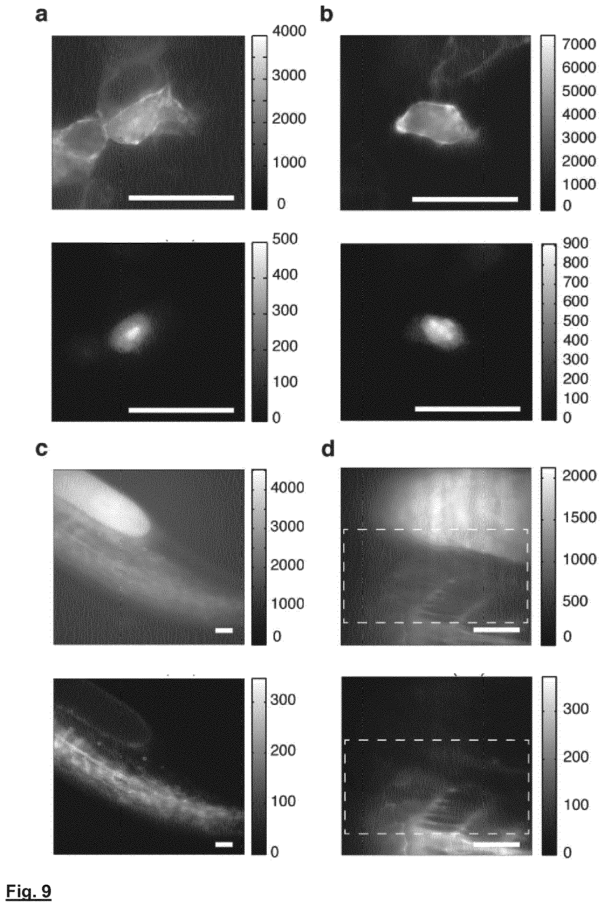

La figure 9 illustre l'application d'un procédé selon un mode de réalisation de l'invention à l'imagerie sélective de matériel biologique exprimant la protéine fluorescente réversiblement convertible « Dronpa-3 ». Les panneaux « a » et « b » de la figure 9 illustrent l'imagerie sélective de « Dronpa-3 » exprimée dans des cellules de mammifère. Chaque panneau présente deux images, l'une correspondant à une photographie prise en épifluorescence et l'autre correspondant à une photographie prise en imagerie sélective de cellules HEK293 exprimant à la fois « Dronpa-3 », qui est une espèce fluorescente réversiblement photoconvertible, dans le noyau, et « EGFP », qui n'est pas une espèce fluorescente réversiblement photoconvertible, sur leur membrane. Le panneau « a » présente une cellule fixée tandis que le panneau « b » présente une cellule vivante dont l'image est prise après une période de modulation du signal lumineux. La barre d'échelle représente 50 $\mu$m.

Les panneaux « c » et « d » illustrent l'imagerie sélective de « Dronpa-3 » dans des embryons de poisson zèbre pris 24 heures après fécondation, et exprimant « lifeact-Dronpa-3 », spécifique à l'actine. De la même manière, chaque panneau présente deux images, l'une correspondant à une photographie prise en épifluorescence et l'autre correspondant à une photographie prise en imagerie sélective Le panneau « c » présente une image dont la modulation de l'amplitude d'éclairage est sinusoïdale et de forte amplitude ($\alpha$ = 90%), et dont les paramètres de contrôle sont réglés sur la résonance de « Dronpa-3 ». Le panneau « d » présente une image dont la modulation de l'amplitude d'éclairage est sous forme de créneaux et de forte amplitude ($\alpha$ = 90%) et dont les paramètres de contrôle sont réglés sur la résonance de « Dronpa-3 ». Le panneau « c » présente des images d'épifluorescence, tandis que le panneau « d » présente des images acquises en microscopie par illumination en plan simple (*Single Plane illumination Microscopy*, SPIM). Dans le panneau « d », le rectangle blanc en pointillés indique la partie la plus fine du plan de lumière d'excitation. L'imagerie sélective permet ici d'observer le réseau d'actine de manière plus spécifique qu'en épifluorescence. Les images des panneaux « a », « b » et « c » de la figure 9 sont prises à 37°C et l'image du panneau « d » est prise à 20°C. La barre d'échelle représente 50 $\mu$m.

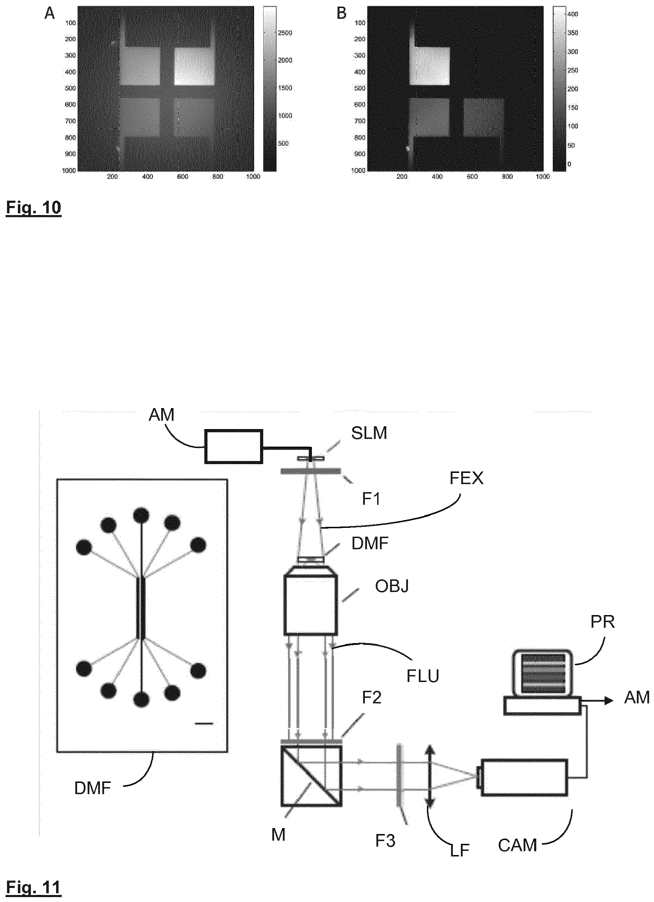

Une autre validation, illustrée par la figure 10 utilise, en tant qu'espèce fluorescente réversiblement photoconvertible, la protéine « Dronpa-2 » qui est excitée par deux faisceaux de lumière distincts, aux longueurs d'ondes sensiblement monochromatiques de 480 nm et 405 nm. L'un des faisceaux ($\lambda$ = 405 $nm$) éclaire à intensité constante et l'autre ($\lambda$ = 480 $nm$) avec une modulation d'intensité de forte amplitude.

Un dispositif microfluidique comprenant quatre chambres carrées (longueur x largeur x épaisseur = 400 x 400 x 20 $\mu$m$^3$) a été utilisé comme échantillon ; les chambres 1 à 4 du dispositif ont été remplies avec les solutions suivantes :

1 : (Dronpa-2 20 $\mu$M, BSA 100 $\mu$M) (en haut à gauche) ;
2 : Fluorescéine 1 $\mu$M seule (en haut à droite) ;
3 : (Dronpa-2 10 $\mu$M, BSA 100 $\mu$M) (en bas à gauche) ;
4 : (Dronpa-2 5 $\mu$M, BSA 100 $\mu$M) (en bas à droite);

Le système Dronpa-2 constitue l'espèce cible et la Fluorescéine un fluorophore interférent.

Le panneau « A » de la figure 10 montre une image de fluorescence de ce dispositif microfluidique acquise, au moyen d'un microscope, en présence d'un éclairage constant. Le panneau « B » correspond au cas d'une détection en quadrature, dans des conditions optimales pour la détection de l'espèce cible.

On peut observer sur le panneau « B » que les intensités de fluorescence $I^{out}_{F,norm}$, mesurées conformément à l'invention, sont sensiblement proportionnelles aux concentrations de Dronpa-2 : un rapport 4 et 2 pour les chambres 1 et 3 comparé à la chambre 4. On observe également que l'intensité de fluorescence $I^{out}_{F,norm}$ est proche de zéro dans le carré contenant uniquement la Fluorescéine.

Bien entendu, d'autres espèces fluorescentes réversiblement photoconvertibles que celles mentionnées cidessus peuvent être utilisées pour mettre en oeuvre l'invention.

La figure 11 illustre un appareil pour la mise en oeuvre d'un procédé selon un mode de réalisation de l'invention, du type utilisé pour la réalisation de certaines des validations expérimentales décrites ci-dessus. Un tel appareil, illustré à titre d'exemple non limitatif, comprend une source de lumière SLM constituée par une barre de diodes électroluminescentes, alimentée par une source d'alimentation AM. La modulation du faisceau lumineux d'excitation FEX généré par ladite source est obtenue par modulation de la puissance électrique d'alimentation. L'émission des diodes électroluminescentes étant à bande large, le faisceau FEX est filtré par un premier filtre optique F1, avant d'être dirigé sur un échantillon, constitué en l'espèce par un dispositif microfluidique DMF. L'échantillon ainsi éclairé est observé, par sa face arrière, par un objectif OBJ qui prélève le rayonnement de fluorescence et le focalise en un faisceau FLU. Ce dernier est filtré (filtres F2, F3) et dirigé, par l'intermédiaire d'un miroir M et d'une lentille LF, sur une caméra CAM. Un processeur PR (en fait, un ordinateur programmé de manière opportune) pilote la source d'alimentation AM et la caméra CAM de manière à effectuer une détection en quadrature telle que décrite plus haut. Pour effectuer une simple détection ou un titrage, sans imagerie, la caméra CAM peut être remplacée par un capteur ponctuel de lumière.

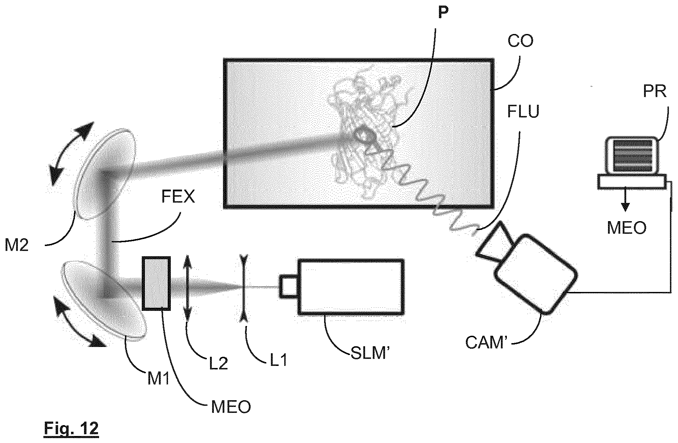

La figure 12 illustre un appareil pour la mise en oeuvre d'un procédé selon un autre mode de réalisation de l'invention, permettant de détecter à distance des sondes fluorescentes réversiblement photoconvertibles dans l'environnement. Dans une telle application, la grande sélectivité rendue possible par la détection en quadrature est nécessaire pour faire émerger le signal utile du fond très intense constitué par la lumière ambiante.

Dans l'appareil de la figure 12, la source de lumière SLM' est un oscillateur laser fonctionnant en régime continu. Le faisceau d'excitation FEX est expansé et collimaté par deux lentilles L1, L2, puis modulé par un modulateur électro-optique MEO. Deux miroirs orientables M1 et M2 sont utilisés pour balayer une cible à observer CO (une surface du sol ou d'un plan d'eau), contenant au moins une espèce fluorescente réversiblement photoconvertible P (Dronpa, par exemple). Le rayonnement de fluorescence FLU est collecté par un objectif d'une caméra CAM', qui acquiert une image de la cible. Comme dans le cas précédent, un processeur PR pilote le modulateur MEO et la caméra CAM' de manière à effectuer une détection en quadrature telle que décrite plus haut.

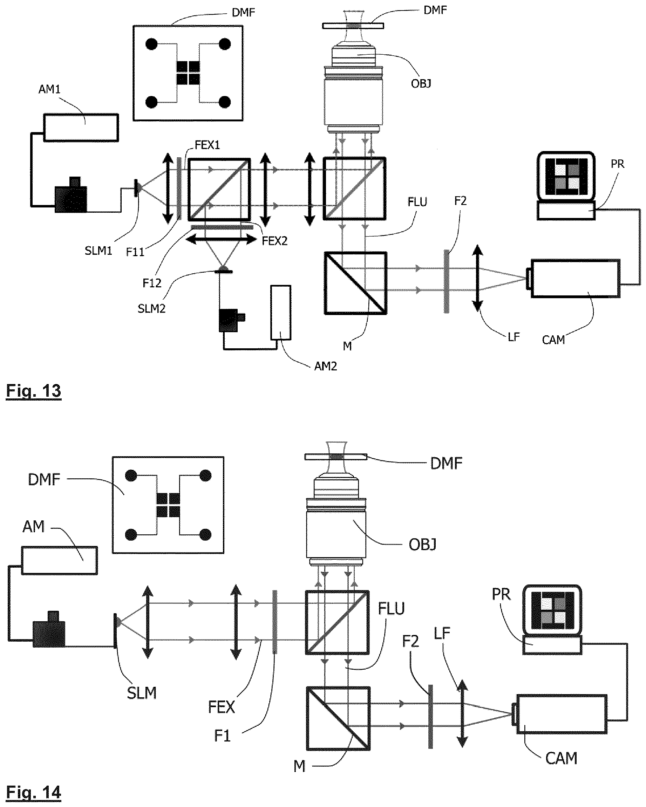

La figure 13 illustre un appareil pour la mise en oeuvre d'un procédé selon un mode de réalisation de l'invention, du type utilisé pour la réalisation de certaines des validations expérimentales décrites ci-dessus. Un tel appareil, illustré à titre d'exemple non limitatif, comprend deux sources de lumière SLM1 et SLM2 constituées par deux barres de diodes électroluminescentes. La source de lumière SLM1 est alimentée par une source d'alimentation AM1 et la source de lumière SLM2 est alimentée par une source d'alimentation AM2. La modulation du faisceau lumineux d'excitation FEX1 généré par ladite source est obtenue par modulation de la puissance électrique d'alimentation. L'émission des diodes électroluminescentes étant à bande large, les faisceaux FEX1 et FEX2 sont filtrés par deux filtres optiques F11 et F12, avant d'être dirigés sur un échantillon, constitué en l'espèce par un dispositif microfluidique DMF. L'échantillon ainsi éclairé est observé, par sa face arrière, par un objectif OBJ qui prélève le rayonnement de fluorescence et le focalise en un faisceau FLU. Ce dernier est filtré (filtre F2) et dirigé, par l'intermédiaire d'un miroir M et d'une lentille LF, sur une caméra CAM. Un processeur PR (en fait, un ordinateur programmé de manière opportune) pilote les sources d'alimentation AM1, AM2 et la caméra CAM de manière à effectuer une détection en quadrature telle que décrite plus haut. Pour effectuer une simple détection ou un titrage, sans imagerie, la caméra CAM peut être remplacée par un capteur ponctuel de lumière.

La figure 14 illustre un appareil pour la mise en oeuvre d'un procédé selon un mode de réalisation de l'invention, du type utilisé pour la réalisation de certaines des validations expérimentales décrites ci-dessus. Un tel appareil, illustré à titre d'exemple non limitatif, comprend une source de lumière SLM constituée par une barre de diodes électroluminescentes, alimentée par une source d'alimentation AM. La modulation du faisceau lumineux d'excitation FEX généré par ladite source est obtenue par modulation de la puissance électrique d'alimentation. L'émission des diodes électroluminescentes étant à bande large, le faisceau FEX est filtré par un premier filtre optique F1, avant d'être dirigé sur un échantillon, constitué en l'espèce par un dispositif microfluidique DMF. L'échantillon ainsi éclairé est observé par un objectif OBJ qui prélève le rayonnement de fluorescence et le focalise en un faisceau FLU. Ce dernier est filtré (filtre F2) et dirigé, par l'intermédiaire d'un miroir M et d'une lentille LF, sur une caméra CAM. Un processeur PR (en fait, un ordinateur programmé de manière opportune) pilote la source d'alimentation AM et la caméra CAM de manière à effectuer une détection en quadrature telle que décrite plus haut. Pour effectuer une simple détection ou un titrage, sans imagerie, la caméra CAM peut être remplacée par un capteur ponctuel de lumière.

L'invention a été décrite en considérant le cas d'une espèce réversiblement photoconvertible émettant un rayonnement de fluorescence. Toutefois, le procédé de détection en quadrature qui vient d'être décrit peut également exploiter toute autre observable spectroscopique permettant de distinguer les deux états d'une espèce réversiblement photoconvertible. A titre d'exemple non limitatif on peut considérer l'absorbance (en particulier en régime de faible absorption, où la loi de Beer-Lambert peut être linéarisée) ; dans ce cas, au lieu de détecter un rayonnement de fluorescence on détecte une intensité lumineuse transmise qui est rapportée à une intensité lumineuse incidente, puis on détermine l'amplitude de sa composante en quadrature de phase. Un autre exemple est constitué par la reflectance. Les équations 19 - 21 et 58 - 59, qui définissent les conditions d'éclairage optimales, s'appliquent quelle que soit l'observable spectroscopique considérée.

**Revendications**

1. Procédé de détection d'au moins une espèce fluorescente réversiblement photoconvertible (P), comportant les étapes suivantes :

   a) éclairer un échantillon contenant ladite ou au moins une dite espèce fluorescente réversiblement photoconvertible avec une lumière d'éclairage (FEX) modulée périodiquement ; et
   b) détecter un rayonnement de fluorescence (FLU) émis par ledit échantillon ainsi éclairé ;

   **caractérisé en ce qu'**il comprend également l'étape suivante :

   c) extraire l'amplitude ($I_F^{out}$) de la composante de l'intensité dudit rayonnement de fluorescence présentant la même périodicité que ladite lumière d'éclairage modulée périodiquement et en quadrature de phase par rapport à elle ;

   et **en ce que** l'intensité moyenne de ladite lumière d'éclairage et sa fréquence de modulation sont choisies de manière à maximiser ladite amplitude de la composante d'intensité dudit rayonnement de fluorescence.

2. Procédé selon la revendication 1 dans lequel au moins une dite espèce fluorescente réversiblement photoconvertible (P) présente un premier et un deuxième état chimique, au moins un desdits états étant fluorescent, ladite ou chaque dite espèce fluorescente réversiblement photoconvertible (P) étant susceptible d'être convertie dudit premier état audit deuxième état par une première réaction photo-induite, puis de retourner audit premier état à la fois par une réaction thermo-induite et par une seconde réaction photo-induite, et dans lequel ladite lumière d'éclairage présente une intensité moyenne $I^0$ et est modulée à une fréquence f avec :

$$I^0 = \frac{k_{21}^{\Delta}}{\sigma_{12} + \sigma_{21}}$$

$$f = k_{21}^{\Delta}/\pi$$

où :

- $\sigma_{12}I^0$ et $\sigma_{21}I^0$ sont, respectivement, les constantes cinétiques de ladite première réaction photo-induite de ladite espèce fluorescente et de ladite seconde réaction photo-induite de ladite espèce fluorescente; et

- $k_{21}^{\Delta}$ est la constante cinétique de ladite réaction thermo-induite de ladite espèce fluorescente.

3. Procédé selon l'une des revendications précédentes dans lequel, lors de ladite étape a), ledit échantillon est éclairé par une lumière d'éclairage sensiblement monochromatique.

4. Procédé selon la revendication 1, dont ladite lumière d'éclairage (FEX) comprend une première lumière d'éclairage sensiblement monochromatique (FEX1) de longueur d'onde $\lambda_1$ et d'une seconde lumière d'éclairage sensiblement monochromatique (FEX2) de longueur d'onde $\lambda_2$, différente de $\lambda_1$, la première et la seconde dites lumières d'éclairage étant adaptées à induire la photoconversion desdits états d'au moins une dite espèce fluorescente réversiblement photoconvertible (P) et dont au moins la première dite lumière d'éclairage est modulée périodiquement.

5. Procédé selon la revendication 4 dans lequel au moins une dite espèce fluorescente réversiblement photoconvertible (P) présente un premier et un deuxième état chimique, au moins un desdits états étant fluorescent, ladite ou chaque dite espèce fluorescente réversiblement photoconvertible (P) étant susceptible d'être convertie dudit premier état audit deuxième état par une première réaction photo-induite, puis de retourner audit premier état par une seconde réaction photo-induite, et dans lequel ladite première lumière d'éclairage présente une intensité moyenne $I_1^0$ et est modulée à une fréquence f' et ladite seconde lumière d'éclairage présente une intensité sensiblement constante $I_2^0$ avec:

$$\frac{I_2^0}{I_1^0} = \frac{\sigma_{12,1} + \sigma_{21,1}}{\sigma_{12,2} + \sigma_{21,2}}$$

$$\frac{f'}{I_1^0} = \left(\sigma_{12,1} + \sigma_{21,1}\right)/\pi$$

où:

- $\sigma_{12,1}I_1^0$ et $\sigma_{21,1}I_1^0$ sont, respectivement, les constantes cinétiques de ladite première et de ladite seconde réaction photo-induite par ladite première lumière d'éclairage ; et

- $\sigma_{12,2}I_2^0$ et $\sigma_{21,2}I_2^0$ sont, respectivement, les constantes cinétiques de ladite première et de ladite seconde réaction photo-induite par ladite seconde lumière d'éclairage.

6. Procédé selon l'une des revendications précédentes, dans lequel ledit échantillon contient une pluralité de dites espèces fluorescentes réversiblement photoconvertibles présentant des propriétés dynamiques différentes, lesdites étapes a) à c) étant mises en oeuvre successivement pour la détection d'au moins deux dites espèces fluorescentes réversiblement photoconvertibles.

7. Procédé selon l'une des revendications précédentes dans lequel lesdites étapes b) et c) sont mises en oeuvre par détection synchrone dudit rayonnement de fluorescence.

8. Procédé selon l'une des revendications précédentes dans lequel ledit échantillon contient au moins une autre espèce fluorescente.

9. Procédé selon l'une des revendications précédentes comportant également l'étape suivante :

d) déterminer la concentration de ladite ou d'au moins une dite espèce fluorescente réversiblement photoconvertible à partir de la composante de l'intensité dudit rayonnement de fluorescence extraite lors de ladite étape c).

10. Procédé selon l'une des revendications précédentes dans lequel ladite ou au moins une dite espèce fluorescente réversiblement photoconvertible est choisie parmi :

- une protéine fluorescente photochrome ; et

- un complexe d'une biomolécule avec une sonde fluorogène.

**11.** Procédé selon l'une des revendications précédentes dont l'échantillon peut comporter de la matière biologique.

**12.** Procédé de microscopie à fluorescence mettant en oeuvre un procédé de détection selon l'une des revendications précédentes.

**13.** Procédé de télédétection optique mettant en oeuvre un procédé de détection selon l'une des revendications 1 à 11.

**14.** Procédé selon la revendication précédente dans lequel ledit échantillon peut comprendre un organisme vivant, et dans lequel au moins un élément choisi parmi la présence et la concentration d'une dite espèce fluorescente réversiblement photoconvertible (P) est mesuré à partir de la composante de l'intensité dudit rayonnement de fluorescence extraite lors de ladite étape c) sans effectuer de prélèvement sur ledit organisme vivant.

**15.** Procédé selon l'une des revendications 13 à 14 dans lequel ladite lumière d'éclairage (FEX) est émise selon une direction et dans lequel ladite modulation périodique de ladite lumière d'éclairage (FEX) est mise en oeuvre par une modulation de ladite direction d'émission de ladite lumière d'éclairage (FEX).

**16.** Procédé selon l'une des revendications 2 et 6 à 15 dans lequel ladite lumière d'éclairage (FEX) comprend une partie de la lumière du jour et dans lequel ladite partie de la lumière du jour participe à l'intensité lumineuse reçue par ladite espèce fluorescente réversiblement photoconvertible (P) en restant inférieure ou égale à ladite intensité $I^0$..

## Patentansprüche

**1.** Verfahren zur Erfassung von mindestens einer umkehrbar fotokonvertiblen (P) fluoreszierenden Art, das die folgenden Schritte aufweist:

a) Beleuchten einer Stichprobe, die die oder mindestens eine umkehrbar fotokonvertible fluoreszierende Art enthält mit einem periodisch modulierten Beleuchtungslicht (FEX); und
b) Erfassung einer durch die derart beleuchtete Stichprobe ausgestrahlten Fluoreszenzstrahlung (FLU);

**dadurch gekennzeichnet, dass** es ebenfalls den folgenden Schritt aufweist:

c) Extrahieren der Größe ($I_F^{out}$) der Komponente für die Intensität der Fluoreszenzstrahlung, die dieselbe regelmäßige Periodizität wie das periodisch modulierte Beleuchtungslicht aufweist und ihm gegenüber um 90° phasenverschoben ist;

und dass die mittlere Intensität des Beleuchtungslichts und seine Modulationsfrequenz so gewählt werden, um die Größe der Komponente für die Intensität der Fluoreszenzstrahlung zu maximieren.

**2.** Verfahren nach Anspruch 1 in dem mindestens eine umkehrbar fotokonvertible (P) fluoreszierende Art einen ersten und einen zweiten chemischen Zustand aufweist, wobei mindestens einer der Zustände fluoreszierend ist, wobei die oder jede umkehrbar fotokonvertible (P) fluoreszierende Art geeignet ist, von dem ersten Zustand in den zweiten Zustand durch eine erste fotoinduzierte Reaktion umgewandelt zu werden, um danach zugleich durch eine thermoinduzierte Reaktion und durch eine zweite fotoinduzierte Reaktion zu dem ersten Zustand zurückzukehren, und wobei das Beleuchtungslicht eine mittlere Intensität $I^0$ aufweist und bei einer Frequenz f moduliert wird mit:

$$I^0 = \frac{k_{21}^\Delta}{\sigma_{12} + \sigma_{21}}$$

$$f = k_{21}^\Delta/\pi$$

worin:

- $\sigma_{12}I^0$ und $\sigma_{21}I^0$ jeweils die kinetischen Konstanten der ersten fotoinduzierten Reaktion der fluoreszierenden Art und der zweiten fotoinduzierten Reaktion der fluoreszierenden Art sind; und

- $k_{21}^{\Delta}$ die kinetische Konstante der thermoinduzierten Reaktion der fluoreszierenden Art ist.

3.  Verfahren nach einem der vorhergehenden Ansprüche, in dem während des Schritts a) die Stichprobe durch ein im Wesentlichen monochromatisches Beleuchtungslicht beleuchtet wird.

4.  Verfahren nach Anspruch 1, bei dem das Beleuchtungslicht (FEX) ein deutlich monochromatisches (FEX1) erstes Beleuchtungslicht mit der Wellenlänge $\lambda_1$ und ein deutlich einfarbiges zweites Beleuchtungslicht (FEX2) mit der Wellenlänge $\lambda_2$ aufweist, ungleich $\lambda_1$, wobei das erste und das zweite Beleuchtungslicht angepasst sind, um die Fotoumwandlung der Zustände von mindestens einer umkehrbar fotokonvertiblen (P) fluoreszierenden Art zu induzieren und wobei mindestens das erste Beleuchtungslicht periodisch moduliert wird.

5.  Verfahren nach Anspruch 4, in dem mindestens eine umkehrbar fotokonvertible (P) fluoreszierende Art einen ersten und einen zweiten chemischen Zustand aufweist, wobei mindestens einer der Zustände fluoreszierend ist, wobei die oder jede umkehrbar fotokonvertible (P) fluoreszierende Art geeignet ist, durch eine erste fotoinduzierte Reaktion von dem ersten Zustand in den zweiten Zustand umgewandelt zu werden, und danach durch eine zweite fotoinduzierte Reaktion zu dem ersten Zustand zurückzukehren, und in der das erste Beleuchtungslicht eine mittlere Intensität $I_1^0$ aufweist und mit einer Frequenz f moduliert wird und das zweite Beleuchtungslicht eine im Wesentlichen konstante Intensität $I_2^0$ aufweist mit:

$$\frac{I_2^0}{I_1^0} = \frac{\sigma_{12,1} + \sigma_{21,1}}{\sigma_{12,2} + \sigma_{21,2}}$$

$$\frac{f'}{I_1^0} = (\sigma_{12,1} + \sigma_{21,1})/\pi$$

worin:

- $\sigma_{12,1}I_1^0$ und $\sigma_{21,1}I_1^0$ jeweils die kinetischen Konstanten der ersten und der zweiten durch das erste Beleuchtungslicht fotoinduzierten Reaktion sind; und

- $\sigma_{12,2}I_2^0$ und $\sigma_{21,2}I_2^0$ jeweils die kinetischen Konstanten der ersten und der zweiten durch das zweite Beleuchtungslicht fotoinduzierten Reaktion sind.

6.  Verfahren nach einem der vorhergehenden Ansprüche, in dem die Stichprobe eine Vielzahl der umkehrbar fotokonvertiblen fluoreszierenden Arten enthält, die unterschiedliche dynamische Eigenschaften aufweisen, wobei die Schritte a) bis c) nacheinander für die Erfassung von mindestens zwei umkehrbar fotokonvertiblen fluoreszierenden Arten ausgeführt werden.

7.  Verfahren nach einem der vorhergehenden Ansprüche, in dem die Schritte b) und c) durch synchrone Erfassung der Fluoreszenzstrahlung ausgeführt werden.

8.  Verfahren nach einem der vorhergehenden Ansprüche, in dem die Stichprobe mindestens eine weitere fluoreszierende Art enthält.

9.  Verfahren nach einem der vorhergehenden Ansprüche, das ebenfalls den folgenden Schritt aufweist:

    d) Bestimmen der Konzentration der oder von mindestens einer umkehrbar fotokonvertiblen fluoreszierenden Art anhand der während des Schritts c) extrahierten Komponente der Intensität der Fluoreszenzstrahlung.

10. Verfahren nach einem der vorhergehenden Ansprüche, in dem die oder mindestens eine fotokonvertibel umkehrbare fluoreszierende Art ausgewählt wird aus:

    - einem photochromen fluoreszierenden Protein; und

- einem Komplex eines Biomoleküls mit einer fluorogenen Sonde.

**11.** Verfahren nach einem der vorhergehenden Ansprüche, wobei die Stichprobe biologische Materie aufweisen kann.

**12.** Fluoreszenzmikroskopieverfahren, das ein Erfassungsverfahren nach einem der vorhergehenden Ansprüche ausführt.

**13.** Verfahren optischer Fernerfassung, das ein Erfassungsverfahren nach einem der Ansprüche 1 bis 11 ausführt.

**14.** Verfahren nach dem vorhergehenden Anspruch, in dem die Stichprobe einen lebenden Organismus aufweisen kann und in dem mindestens ein Element, das aus dem Vorkommen und der Konzentration einer umkehrbar fotokonvertiblen (P) fluoreszierenden Art ausgewählt wird, anhand der Komponente für die Intensität der Fluoreszenzstrahlung gemessen wird, die während des Schritts c), ohne eine Probenentnahme von dem lebenden Organismus auszuführen, extrahiert wird.

**15.** Verfahren nach einem der Ansprüche 13 bis 14, in dem das Beleuchtungslicht (FEX) gemäß einer Richtung ausgestrahlt wird und in dem die periodische Modulation des Beleuchtungslichts (FEX) durch eine Modulation der Ausstrahlungsrichtung des Beleuchtungslichts (FEX) ausgeführt wird.

**16.** Verfahren nach einem der Ansprüche 2 und 6 bis 15, in dem das Beleuchtungslicht (FEX) einen Teil des Tageslichts umfasst und in dem der Teil des Tageslichts zu der Helligkeit beiträgt, die durch die umkehrbar fotokonvertible (P) fluoreszierende Art empfangen wird, wobei sie unter oder gleich der Intensität $I^0$ bleibt.

**Claims**

**1.** A method for detecting at least one reversibly photoswitchable fluorescent species (P) comprising the following steps:

a) illuminating a sample containing said or at least one of said reversibly photoswitchable fluorescent species with a periodically modulated illuminating light (FEX); and
b) detecting a fluorescent radiation (FLU) emitted by said duly illuminated sample,

**characterised in that** it further comprises the following step:

c) extracting the amplitude ($I_F^{out}$) of the component of the intensity of said fluorescent radiation having the same periodicity as said periodically modulated illuminating light and in phase quadrature therewith;

and **in that** the average intensity of said illuminating light and its modulation frequency are selected so as to maximise said amplitude of the intensity component of said fluorescent radiation.

**2.** The method according to claim 1, wherein at least one of said reversibly photoswitchable fluorescent species (P) has a first and a second chemical state, at least one of said states being fluorescent, said or each of said reversibly photoswitchable fluorescent species (P) being able to be converted from said first state to said second state by a first photo-induced reaction, then to return to said first state both by a thermo-induced reaction and by a second photo-induced reaction, and wherein said illuminating light has an average intensity $I^0$ and is modulated at a frequency f, with:

$$I^0 = \frac{k_{21}^{\Delta}}{\sigma_{12} + \sigma_{21}}$$

$$f = k_{21}^{\Delta}/\pi$$

where:

- $\sigma_{12}I^0$ and $\sigma_{21}I^0$ are, respectively, the kinetic constants of said first photo-induced reaction of said fluorescent species and of said second photo-induced reaction of said fluorescent species; and

- $k_{21}^{\Delta}$ is the kinetic constant of said thermo-induced reaction of said fluorescent species.

3. The method according to any one of the preceding claims, wherein, during said step a), said sample is illuminated by a substantially monochromatic illuminating light.

4. The method according to claim 1, wherein said illuminating light (FEX) comprises a first substantially monochromatic illuminating light (FEX1) of wavelength $\lambda_1$ and a second substantially monochromatic illuminating light (FEX2) of wavelength $\lambda_2$, different to $\lambda_1$, the first and the second of said illuminating lights being adapted to induce the photoswitching of said states of at least one of said reversibly photoswitchable fluorescent species (P) and for which at least the first of said illuminating lights is periodically modulated.

5. The method according to claim 4, wherein at least one of said reversibly photoswitchable fluorescent species (P) has a first and a second chemical state, at least one of said states being fluorescent, said or each of said reversibly photoswitchable fluorescent species (P) being able to be converted from said first state to said second state by a first photo-induced reaction, then to return to said first state by a second photo-induced reaction, and wherein said first illuminating light has an average intensity $I_1^0$ and is modulated at a frequency f' and said second illuminating light has a substantially constant intensity $I_2^0$, with:

$$\frac{I_2^0}{I_1^0} = \frac{\sigma_{12.1} + \sigma_{21.1}}{\sigma_{12.2} + \sigma_{21.2}}$$

$$\frac{f'}{I_1^0} = (\sigma_{12.1} + \sigma_{21.1})/\pi$$

where:
- $\sigma_{12.1}I_1^0$ and $\sigma_{21.1}I_1^0$ are, respectively, the kinetic constants of said first and said second reaction photo-induced by said first illuminating light; and
- $\sigma_{12.2}I_2^0$ and $\sigma_{21.2}I_2^0$ are, respectively, the kinetic constants of said first and said second reaction photo-induced by said second illuminating light.

6. The method according to any one of the preceding claims, wherein said sample contains a plurality of said reversibly photoswitchable fluorescent species having different dynamic properties, said steps a) to c) being successively implemented for detecting at least two of said reversibly photoswitchable fluorescent species.

7. The method according to any one of the preceding claims, wherein said steps b) and c) are implemented by synchronous detection of said fluorescent radiation.

8. The method according to any one of the preceding claims, wherein said sample contains at least one other fluorescent species.

9. The method according to any one of the preceding claims, further comprising the following step:

d) determining the concentration of said or of at least one of said reversibly photoswitchable fluorescent species on the basis of the component of the intensity of said fluorescent radiation extracted during said step c).

10. The method according to any one of the preceding claims, wherein said or at least one of said reversibly photoswitchable fluorescent species is selected from:

- a photochromatic fluorescent protein; and
- a complex of a biomolecule with a fluorogenic probe.

11. The method according to any one of the preceding claims, wherein the sample can comprise biological material.

12. A fluorescence microscopy method implementing a detection method according to any one of the preceding claims.

13. An optical remote sensing method implementing a detection method according to any one of claims 1 to 11.

14. The method according to the preceding claim, wherein said sample can comprise a living organism and wherein at least one element selected from the presence and the concentration of one of said reversibly photoswitchable fluorescent species (P) is measured on the basis of the component of the intensity of said fluorescent radiation extracted during said step c) without taking a sample on said living organism.

15. The method according to any one of claims 13 to 14, wherein said illuminating light (FEX) is emitted in one direction and wherein said periodic modulation of said illuminating light (FEX) is implemented by a modulation of said emission direction of said illuminating light (FEX).

16. The method according to any one of claims 2 and 6 to 15, wherein said illuminating light (FEX) comprises a part of daylight and wherein said part of daylight participates in the light intensity received by said reversibly photoswitchable fluorescent species (P) while remaining less than or equal to said intensity $I^0$.

**Fig. 1**

**Fig. 2A**

**Fig. 2B**

**Fig. 3**

$P_{titration}^{0}$

$P_{titration}^{1cos}$

**Fig. 4**

$\left|\dfrac{1^{Iout}}{P_{tot}}\right|$

$\log_{10}\left(\dfrac{\omega}{I_1^0}\right)$

$\log_{10}\left(\dfrac{I_2^0}{I_1^0}\right)$

**Fig. 5A**

B

$\left|\dfrac{1^{1out}}{P_{tot}}\right|$

$\log_{10}(\dfrac{\omega}{I_1^0})$

$\log_{10}(\dfrac{I_2^0}{I_1^0})$

Fig . 5B

Fig. 6

**Fig. 7**

**Fig. 8**

**Fig. 9**

**Fig. 10**

**Fig. 11**

**Fig. 12**

**Fig. 13**

**Fig. 14**

EP 3 071 948 B1

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Littérature non-brevet citée dans la description**

- **G. MARRIOTT et al.** Optical lock-in detection imaging microscopy for contrast-enhanced imaging in living cell. *PNAS,* 18 Novembre 2008, vol. 105 (46), 17789-17794

- **CH. I. RICHARDS et al.** Synchronously Amplified Fluorescence Image Recovery (SAFIRe). *J. Phys. Chem. B,* 2010, vol. 114, 660-665
- **Q. WEI ; A. WEI.** Optical Imaging with Dynamic Contrast Agents. *Chem. Eur. J.,* vol. 17, 1080-1091